# EUROPEAN PATENT SPECIFICATION

(11) **EP 1 420 769 B1**
(45) Date of publication and mention of the grant of the patent: **11.06.2008**
(21) Application number: 02707001.0
(22) Date of filing: 25.03.2002
(51) Int. Cl.: A61K 31/00, A61K 31/57, A61K 31/5685, A61P 3/06, A61P 3/10, A61P 3/04, A61P 5/46, A61P 5/50, A61P 9/00, A61P 9/10, A61P 1/16, A61P 29/00, A61K 31/56, A61K 31/573, A61K 31/225, C12N 15/11, A61K 39/395, A61K 31/192

(54) **LIPID PROFILE MODULATION WITH STEROIDS AND PPAR ALPHA AGONISTS**
LIPIDSPIEGEL MODULATION MIT STEROIDEN UND PPAR-ALPHA AGONISTEN
MODULATION D'UN PROFILE LIPIDIQUE AVEC DES STEROIDES ET DES AGONISTES DE PPAR ALPHA

(30) Priority: 23.03.2001 GB 0107383
(43) Date of publication of application: 26.05.2004
(73) Proprietor: THE UNIVERSITY OF EDINBURGH, South Bridge, Edinburgh EH8 9YL (GB)
(72) Inventor: MORTON, Nicholas M., University of Edinburgh, Edinburgh EH4 2XU (GB); SECKL, Jonathan Robert, University of Edinburgh, Edinburgh EH16 4TJ (GB); WALKER, Brian Robert, University of Edinburgh, Edinburgh EH16 4TJ (GB); ANDREW, Ruth, Universtiy of Edinburgh, Edinburgh EH4 2XU (GB)
(74) Representative: Harding, Charles Thomas
(86) International application number: PCT/GB2002/001457
(87) International publication number: WO 2002/076435

(56) References cited:
- EP-A- 1 157 695
- WO-A-01/90092
- WO-A-02/02797
- WO-A-97/07789
- WO-A-02/072084
- US-A- 4 518 595
- US-A- 4 920 115
- WALKER B R: "CORTISOL SECRETION AND SENSITIVITY IN INSULIN RESISTANCE" JOURNAL OF ENDOCRINOLOGY, BRISTOL, GB, vol. 160, no. SUPPL, March 1999 (1999-03), page S37 XP008015770 ISSN: 0022-0795
- HERMANOWSKI-VOSATKA A ET AL: "PPARALPHA AGONISTS REDUCE THE EXPRESSION OF 11BETAHSD1 IN THE LIVER" JOURNAL OF ENDOCRINOLOGY, BRISTOL, GB, vol. 164, March 2000 (2000-03), page 283 XP008015933 ISSN: 0022-0795
- HULT M ET AL: "Selective inhibition of human type 1 11beta-hydroxysteroid dehydrogenase by synthetic steroids and xenobiotics" FEBS LETTERS, ELSEVIER SCIENCE PUBLISHERS, AMSTERDAM, NL, vol. 441, no. 1, 11 December 1998 (1998-12-11), pages 25-28, XP004258864 ISSN: 0014-5793
- DATABASE CAPLUS [Online] CHEMICAL ABSTRACTS SERVICE, COLUMBUS, OHIO, US; 1982 SUETINA I V: "[Changes in the lipid metabolic indices as affected by glycyrrhenate in experimental hyperlipidemia]" retrieved from STN, accession no. 1982:607991 Database accession no. 97:207991 XP002241943 & NAUCHNYE DOKLADY VYSSHEI SHKOLY. BIOLOGICHESKIE NAUKI. USSR 1982, no. 8, 1982, pages 47-50, ISSN: 0470-4606
- DATABASE CAPLUS [Online] CHEMICAL ABSTRACTS SERVICE, COLUMBUS, OHIO, US; 1984 VASILENKO YU K ET AL: "COMPARATIVE EVALUATION OF THE HYPOLIPIDEMIC EFFECT OF SOME LICORICE PREPARATIONS" retrieved from STN, accession no. 1985:553804 Database accession no. 103:153804 XP002241944 & IZVESTIYA SEVERO-KAVKAZSKOGO NAUCHNOGO TSENTRA VYSSHEI SHKOLY, no. 4, 1984, pages 83-87, 1984 (RECD. 1985) ISSN: 0321-3005
- WHORWOOD C B ET AL: "REGULATION OF GLUCOCORTICOID RECEPTOR ALPHA AND BETA ISOFORMS AND TYPE I 11BETA-HYDROXYSTEROID DEHYDROGENASE EXPRESSION IN HUMAN SKELETAL MUSCLE CELLS: A KEY ROLE IN THE PATHOGENESIS OF INSULIN RESISTANCE?" JOURNAL OF CLINICAL ENDOCRINOLOGY AND METABOLISM, NEW YORK, NY, US, vol. 86, no. 5, 2001, pages 2296-2308, XP001149824 ISSN: 0021-972X
- MCINTOSH M ET AL: "OPPOSING ACTIONS OF DEHYDROEPIANDROSTERONE AND CORTICOSTERONE IN RATS (44405)" PROCEEDINGS OF THE SOCIETY FOR EXPERIMENTAL BIOLOGY & MEDICINE, ACADEMIC PRESS INC. NEW YORK, US, vol. 221, no. 3, July 1999 (1999-07), pages 198-206, XP001148856 ISSN: 0037-9727
- NESTLER J E ET AL: "DEHYDROEPIANDROSTERONE: THE MISSING LINK BETWEEN HYPERINSULINEMIA AND ATHEROSCLEROSIS?" FASEB JOURNAL, FED. OF AMERICAN SOC. FOR EXPERIMENTAL BIOLOGY, BETHESDA, MD, US, vol. 6, no. 12, September 1992 (1992-09), pages 3073-3075, XP001148845 ISSN: 0892-6638
- RICHARDS R J ET AL: "SERUM LEPTIN, LIPIDS, FREE FATTY ACIDS, AND FAT PADS IN LONG-TERM DEHYDROEPIANDROSTERONE-TREATED ZUCKER RATS" PROCEEDINGS OF THE SOCIETY FOR EXPERIMENTAL BIOLOGY & MEDICINE, WILLIAMS AND WILKINS, XX, vol. 223, no. 3, March 2000 (2000-03), pages 258-262, XP008015883 ISSN: 0037-9727
- DATABASE CAPLUS [Online] CHEMICAL ABSTRACTS SERVICE, COLUMBUS, OHIO, US; July 2001 (2001-07) SCHWARTZ A G ET AL: "Potential therapeutic use of dehydroepiandrosterone and structural analogs." retrieved from STN, accession no. 2001:528848 Database accession no. 135:267339 XP002241945 & DIABETES TECHNOLOGY & THERAPEUTICS. UNITED STATES 2001 SUMMER, vol. 3, no. 2, July 2001 (2001-07), pages 221-224, ISSN: 1520-9156
- DATABASE MEDLINE [Online] US NATIONAL LIBRARY OF MEDICINE (NLM), BETHESDA, MD, US; 29 December 1995 (1995-12-29) MILEWICH L ET AL: "Pleotropic effects of dietary DHEA." Database accession no. NLM8597455 XP002241946 & ANNALS OF THE NEW YORK ACADEMY OF SCIENCES. UNITED STATES 29 DEC 1995, vol. 774, 29 December 1995 (1995-12-29), pages 149-170, ISSN: 0077-8923
- TCHERNOF A ET AL: "Relationships between endogenous steroid hormone, sex hormone-binding globulin and lipoprotein levels in men: contribution of visceral obesity, insulin levels and other metabolic variables." ATHEROSCLEROSIS. IRELAND SEP 1997, vol. 133, no. 2, September 1997 (1997-09), pages 235-244, XP002241940 ISSN: 0021-9150
- ALEXANDERSEN P ET AL: "NATURAL ANDROGENS INHIBIT MALE ATHEROSCLEROSIS A STUDY IN CASTRATED, CHOLESTEROL-FED RABBITS" CIRCULATION RESEARCH, GRUNE AND STRATTON, BALTIMORE, US, vol. 84, no. 7, 16 April 1999 (1999-04-16), pages 813-819, XP001149840 ISSN: 0009-7330
- SMITH K J ET AL: "Peroxisomal proliferator-activated ligand therapy for HIV lipodystrophy." CLINICAL AND EXPERIMENTAL DERMATOLOGY. ENGLAND MAR 2001, vol. 26, no. 2, March 2001 (2001-03), pages 155-161, XP002241941 ISSN: 0307-6938
- KURZMAN I D ET AL: "THE EFFECT OF DEHYDROEPIANDROSTERONE COMBINED WITH A LOW-FAT DIET IN SPONTANEOUSLY OBESE DOGS: A CLINICAL TRIAL" OBESITY RESEARCH, BATON ROUGE, LA,, US, vol. 6, no. 1, January 1998 (1998-01), pages 20-28, XP008015881 ISSN: 1071-7323
- RAO M S ET AL: "DEHYDROEPIANDROSTERONE INHIBITS DNA SYNTHESIS OF RAT HEPATOCYTES INDUCED BY PARTIAL HEPATECTOMY OF MITOGEN (CIPROFIBRATE)" CELL PROLIFERATION, OXFORD, GB, vol. 30, no. 1, 1997, pages 1-5, XP008015877 ISSN: 0960-7722
- HAFFA A L M ET AL: "HYPOCHOLESTEROLEMIC EFFECT OF EXOGENOUS DEHYDROEPIANDROSTERONE ADMINISTRATION IN THE RHESUS MONKEY" IN VIVO - INTERNATIONAL JOURNAL OF IN VIVO RESEARCH, XX, GB, vol. 8, no. 6, November 1994 (1994-11), pages 993-997, XP008015868 ISSN: 0258-851X
- CHANCE D S ET AL: "INVERSE RELATIONSHIP BETWEEN PEROXISOMAL AND MITOCHONDRIAL BETA-OXIDATION IN HEPG2 CELLS TREATED WITH DEHYDROEPIANDROSTERONE AND CLOFIBRIC ACID" PROCEEDINGS OF THE SOCIETY FOR EXPERIMENTAL BIOLOGY & MEDICINE, ACADEMIC PRESS INC. NEW YORK, US, vol. 205, no. 4, April 1995 (1995-04), pages 378-384, XP001148841 ISSN: 0037-9727
- ALEXANDERSEN P ET AL: "The relationship of natural androgens to coronary heart disease in males: a review." ATHEROSCLEROSIS. IRELAND 23 AUG 1996, vol. 125, no. 1, 23 August 1996 (1996-08-23), pages 1-13, XP002241942 ISSN: 0021-9150
- GORDON G B ET AL: "REDUCTION OF ATHEROSCLEROSIS BY ADMINISTRATION OF DEHYDROEPIANDROSTERONE. A STUDY IN THE HYPERCHOLESTEROLEMIC NEW ZEALAND WHITE RABBIT WITH AORTIC INTIMAL INJURY" JOURNAL OF CLINICAL INVESTIGATION, NEW YORK, NY, US, vol. 82, no. 2, 1 August 1988 (1988-08-01), pages 712-720, XP002049299 ISSN: 0021-9738
- DATABASE MEDLINE [Online] US NATIONAL LIBRARY OF MEDICINE (NLM), BETHESDA, MD, US; March 1989 (1989-03) ARAD Y ET AL: "Dehydroepiandrosterone feeding prevents aortic fatty streak formation and cholesterol accumulation in cholesterol-fed rabbit." Database accession no. NLM2522296 XP002241947 & ARTERIOSCLEROSIS (DALLAS, TEX.) UNITED STATES 1989 MAR-APR, vol. 9, no. 2, March 1989 (1989-03), pages 159-166, ISSN: 0276-5047
- DATABASE CAPLUS [Online] CHEMICAL ABSTRACTS SERVICE, COLUMBUS, OHIO, US; 1968 ADLERCREUTZ H ET AL: "Studies in plasma neutral steroid sulphates, urinary 11-deoxy-17-ketosteroids and estrogens, and plasma lipids in patients with hypercholesterolemia or hyperlipemia." retrieved from STN, accession no. 1968:465726 Database accession no. 69:65726 XP002241948 & ANNALES MEDICINAE EXPERIMENTALIS ET BIOLOGIAE FENNIAE. FINLAND 1968, vol. 46, no. 2, 1968, pages 165-171, ISSN: 0003-4479
- WALKER B R: "ABNORMAL GLUCOCORTICOID ACTIVITY IN SUBJECTS WITH RISK FACTORS FOR CARDIOVASCULAR DISEASE" ENDROCRINE RESEARCH, MARCEL DEKKER, NEW YORK, NY, US, vol. 22, no. 4, November 1996 (1996-11), pages 701-708, XP008023578 ISSN: 0743-5800
- MORTON N M ET AL: "IMPROVED LIPID AND LIPOPROTEIN PROFILE, HEPATIC INSULIN SENSITIVITY, AND GLUCOSE TOLERANCE IN 11BETA-HYDROXYSTEROID DEHYDROGENASE TYPE 1 NULL MICE" JOURNAL OF BIOLOGICAL CHEMISTRY, AMERICAN SOCIETY OF BIOLOGICAL CHEMISTS, BALTIMORE, MD, US, vol. 276, no. 44, 2 November 2001 (2001-11-02), pages 41293-41300, XP001149394 ISSN: 0021-9258
- BERGER J ET AL: "PEROXISOME PROLIFERATOR-ACTIVATED RECEPTOR-GAMMA LIGANDS INHIBIT ADIPOCYTE 11BETA-HYDROXYSTEROID DEHYDROGENASE TYPE 1 EXPRESSION AND ACTIVITY" JOURNAL OF BIOLOGICAL CHEMISTRY, AMERICAN SOCIETY OF BIOLOGICAL CHEMISTS, BALTIMORE, MD, US, vol. 276, no. 16, 20 April 2001 (2001-04-20), pages 12629-12635, XP001149395 ISSN: 0021-9258
- HERMANOWSKI-VOSATKA A ET AL: "PPARALPHA AGONISTS REDUCE 11BETA-HYDROXYSTEROID DEHYDROGENASE TYPE 1 IN THE LIVER" BIOCHEMICAL AND BIOPHYSICAL RESEARCH COMMUNICATIONS, ACADEMIC PRESS INC. ORLANDO, FL, US, vol. 279, no. 2, 20 December 2000 (2000-12-20), pages 330-336, XP001149396 ISSN: 0006-291X
- WALKER B R ET AL: "CARBENOXOLONE INCREASES HEPATIC INSULIN SENSITIVITY IN MAN: A NOVELROLE FOR 11-OXOSTEROID REDUCTASE IN ENHANCING GLUCOCORTICOID RECEPTOR ACTIVATION" JOURNAL OF CLINICAL ENDOCRINOLOGY AND METABOLISM, NEW YORK, NY, US, vol. 80, no. 11, 1 November 1995 (1995-11-01), pages 3155-3159, XP000612666 ISSN: 0021-972X cited in the application
- JAMIESON P M ET AL: "INTERACTIONS BETWEEN OESTRADIOL AND GLUCOCORTICOID REGULATORY EFFECTS ON LIVER-SPECIFIC GLUCOCORTICOID-INDUCIBLE GENES: POSSIBLE EVIDENCE FOR A ROLE OF HEPATIC 11BETA-HYDROXYSTEROID DEHYDROGENASE TYPE 1" JOURNAL OF ENDOCRINOLOGY, BRISTOL, GB, vol. 160, no. 1, January 1999 (1999-01), pages 103-109, XP008015659 ISSN: 0022-0795
- STEWART P M ET AL: "GROWTH HORMONE, INSULIN-LIKE GROWTH FACTOR-I AND THE CORTISOL-CORTISONE SHUTTLE" HORMONE RESEARCH, S. KARGER AG, BASEL, CH, vol. 56, no. SUPPL 1, 2001, pages 1-6, XP008015663 ISSN: 0301-0163
- ANDREWS R C ET AL: "GLUCOCORTICOIDS AND INSULIN RESISTANCE: OLD HORMONES, NEW TARGETS" CLINICAL SCIENCE, BIOCHEMICAL SOCIETY AND THE MEDICAL RESEARCH SOCIETY, LONDON,, GB, vol. 96, no. 5, May 1999 (1999-05), pages 513-523, XP001172422 ISSN: 0143-5221
- DIEDERICH S ET AL: "In the search for specific inhibitors of human 11beta-hydroxysteroid-dehydrogenases (11beta-HSDs): Chenodeoxycholic acid selectively inhibits 11beta-HSD-I" EUROPEAN JOURNAL OF ENDOCRINOLOGY, SCANDINAVIAN UNIVERSITY PRESS, NO, vol. 142, no. 2, February 2000 (2000-02), pages 200-207, XP002226259 ISSN: 0804-4643
- AOKI K ET AL: "DEHYDROEPIANDROSTERONE SUPPRESSES THE ELEVATED HEPATIC GLUCOSE-6-PHOSPHATASE AND FRUCTOSE-1,6-BISPHOSPHATASE ACTIVITIES IN C57BL/KSJ-DB/DB MICE COMPARISON WITH TROGLITAZONE" DIABETES, NEW YORK, NY, US, vol. 48, no. 8, August 1999 (1999-08), pages 1579-1585, XP001150303 ISSN: 0012-1797
- HANSEN P A ET AL: "DHEA PROTECTS AGAINST VISCERAL OBESITY AND MUSCLE INSULIN RESISTANCE IN RATS FED A HIGH-FAT DIET" AMERICAN JOURNAL OF PHYSIOLOGY, AMERICAN PHYSIOLOGICAL SOCIETY, BETHESDA, MD, US, vol. 273, no. 5, PART 2, November 1997 (1997-11), pages R1704-R1708, XP008015768 ISSN: 0002-9513
- WALKER B R ET AL: "INCREASED GLUCORTICOID ACTIVITY IN MEN WITH CARDIOVASCULAR RISK FACTORS" HYPERTENSION, XX, XX, vol. 31, no. 4, April 1998 (1998-04), pages 891-895, XP001149397 ISSN: 0194-911X
- WATSON R R ET AL: "DEHYDROEPIANDROSTERONE AND DISEASES OF AGING" DRUGS AND AGING, ADIS INTERNATIONAL LTD, NZ, vol. 9, no. 4, October 1996 (1996-10), pages 274-291, XP008015849 ISSN: 1170-229X
- MUKASA K ET AL: "Dehydroepiandrosterone (DHEA) ameliorates the insulin sensitivity in older rats." THE JOURNAL OF STEROID BIOCHEMISTRY AND MOLECULAR BIOLOGY. ENGLAND NOV 1998, vol. 67, no. 4, November 1998 (1998-11), pages 355-358, XP002259535 ISSN: 0960-0760
- ABADIE J M: "EFFECTS OF DEHYDROEPIANDROSTERONE ON FATTY ACIDS PROFILES IN ZUCKER RATS: EVIDENCE FOR DECREASING INSULIN RESISTANCE" DISSERTATION ABSTRACTS INTERNATIONAL. B. THE SCIENCE AND ENGINEERING, ANN ARBOR, US, vol. 60, no. 7, 1999, page 3215 XP008015870 ISSN: 0419-4217
- KOTELEVTSEV Y ET AL: "11beta-hydroxysteroid dehydrogenase type 1 knockout mice show attenuated glucocorticoid-inducible responses and resist hyperglycemia on obesity or stress" PROCEEDINGS OF THE NATIONAL ACADEMY OF SCIENCES OF USA, NATIONAL ACADEMY OF SCIENCE. WASHINGTON, US, vol. 94, no. 26, 23 December 1997 (1997-12-23), pages 14924-14929, XP002220014 ISSN: 0027-8424
- ANDREWS R C ET AL: "ABNORMAL GLUCOCORTICOID METABOLISM AND SENSITIVITY IN TYPE 2 DIABETES MELLITUS" JOURNAL OF ENDOCRINOLOGY, BRISTOL, GB, vol. 163, no. SUPPL, November 1999 (1999-11), page 28 XP008015657 ISSN: 0022-0795
- COLEMAN D L ET AL: "Therapeutic effects of dehydroepiandrosterone (DHEA) in diabetic mice." DIABETES. UNITED STATES SEP 1982, vol. 31, no. 9, September 1982 (1982-09), pages 830-833, XP008023566 ISSN: 0012-1797
- ASSAN R ET AL: "Dehydroepiandrosterone (DHEA) for diabetic patients?" EUROPEAN JOURNAL OF ENDOCRINOLOGY / EUROPEAN FEDERATION OF ENDOCRINE SOCIETIES. NORWAY JUL 1996, vol. 135, no. 1, July 1996 (1996-07), pages 37-38, XP008023704 ISSN: 0804-4643
- BUFFINGTON C K ET AL: "Case report: amelioration of insulin resistance in diabetes with dehydroepiandrosterone." THE AMERICAN JOURNAL OF THE MEDICAL SCIENCES. UNITED STATES NOV 1993, vol. 306, no. 5, November 1993 (1993-11), pages 320-324, XP008023722 ISSN: 0002-9629
- HOLMES M C ET AL: "PHENOTYPIC ANALYSIS OF MICE BEARING TARGETED DELETIONS OF 11BETA-HYDROXYSTEROID DEHYDROGENASES 1 AND 2 GENES" MOLECULAR AND CELLULAR ENDOCRINOLOGY, AMSTERDAM, NL, vol. 171, no. 1/2, 22 January 2001 (2001-01-22), pages 15-20, XP001149391 ISSN: 0303-7207
- WALKER B R: "STEROID METABOLISM IN METABOLIC SYNDROME X" BAILLIERE'S BEST PRACTICE AND RESEARCH. CLINICAL ENDOCRINOLOGY AND METABOLISM, BAILLIERE TINDALL, LONDON, GB, vol. 15, no. 1, March 2001 (2001-03), pages 111-122, XP008015671 ISSN: 1521-690X
- LIVINGSTONE D E W ET AL: "MECHANISMS OF DYSREGULATION OF 11BETA-HYDROXYSTEROID DEHYDROGENASE TYPE 1 IN OBESE ZUKER RATS" JOURNAL OF ENDOCRINOLOGY, BRISTOL, GB, vol. 167, no. 3, December 2000 (2000-12), pages 533-539, XP008015658 ISSN: 0022-0795
- DATABASE EMBASE [Online] ELSEVIER SCIENCE PUBLISHERS, AMSTERDAM, NL; 1999 SUZUKI M ET AL: "A close association between insulin resistance and dehydroepiandrosterone sulfate in subjects with essential hypertension" Database accession no. EMB-1999324319 XP002259536 & ENDOCRINE JOURNAL 1999 JAPAN, vol. 46, no. 4, 1999, pages 521-528, ISSN: 0918-8959
- SANDEEP T C ET AL: "PATHOPHYSIOLOGY OF MODULATION OF LOCAL GLUCOCORTICOID LEVELS BY 11BETA-HYDROXYSTEROID DEHYDROGENASES" TRENDS IN ENDOCRINOLOGY AND METABOLISM, ELSEVIER SCIENCE PUBLISHING, NEW YORK, NY, US, vol. 12, no. 10, December 2001 (2001-12), pages 446-453, XP001149392 ISSN: 1043-2760

## Description

### Field of the Invention

The present invention relates to a method for modulating the lipid profile of an individual to obtain an atheroprotective effect. In particular, the invention relates to the modulation of 11β-Hydroxysteroid Dehydrogenase Type 1 (11β-HSD-1) levels for the promotion of atheroprotective lipid profiles.

### Introduction

The metabolic syndrome is emerging as one of the major medical and public health problems both in the United States and worldwide. It is characterised by hypertension, hypertriglyceridaemia, and hyperglycaemia, is exacerbated by obesity, and constitutes a risk factor for coronary heart disease.

Coronary heart disease is a condition that manifests as either heart attack (myocardial infarction), heart failure or chest pain (angina pectoris). It is caused by a narrowing and hardening of the coronary arteries (atherosclerosis). One of the primary features of atherosclerosis is the accumulation of cholesterol within the walls of the coronary arteries. Risk factors for coronary heart disease are the underlying causes of atherosclerosis. There are three major causes of coronary atherosclerosis: elevated LDL cholesterol, cigarette smoking, and the metabolic syndrome. Among these LDL cholesterol is the primary cause of atherosclerosis. When the blood level of LDL is increased, atherosclerosis is initiated and sustained. Cigarette smoking and the metabolic syndrome nevertheless constitute significant risk factors.

The metabolic syndrome is composed of individual risk factors that in aggregate greatly raise the risk for coronary heart disease. The metabolic risk factors that make up this syndrome are high triglycerides, small LDL particles, low HDL cholesterol, high blood pressure, high blood glucose, a tendency for blood clotting (thrombosis), and chronic inflammation. Taken in aggregate, these risk factors accelerate the development of atherosclerosis when they occur in the presence of elevated LDL cholesterol. When LDL-cholesterol levels are very low, the risk factors of the metabolic syndrome may have less effect on atherogenesis; but once LDL levels rise, these other risk factors are believed to become increasingly atherogenic.

Many patients with metabolic syndrome moreover develop type 2 diabetes (adult-onset diabetes). Type 2 diabetes is characterised by a fasting plasma glucose level of 7.0 mmol/l or higher. Most persons with type 2 diabetes have two metabolic abnormalities that raise the blood glucose to the diabetes range. The first abnormality is insulin resistance; the other is a deficiency in production of insulin by the pancreas.

Type 2 diabetes typically develops when insulin resistance is combined with a mild-to-moderate defect in the secretion of insulin. Insulin resistance thus is a disorder in the metabolism of tissues that interferes with the normal action of insulin to promote glucose uptake and utilisation. It usually precedes the development of type 2 diabetes by many years. There is a close connection between insulin resistance and the risk factors of the metabolic syndrome. The nature of this connection is not fully understood. One factor appears to be an overloading of tissues with fats (lipids). Patients with insulin resistance usually have a high level of free fatty acids, which are released from fat tissue (adipose tissue). When excess fatty acids enter muscle, lipid overload occurs, and this induces insulin resistance. Other factors may contribute to insulin resistance, but tissue overload of lipids appears to be a major factor. This overload in various ways seems to engender the coronary risk factors of the metabolic syndrome.

An elevated blood LDL cholesterol level generally is not considered to be an integral component of the metabolic syndrome. Nevertheless, it is a major independent risk factor that must be present before the other components of the metabolic syndrome can come into play as atherogenic factors. In populations around the world in which the various components of the metabolic syndrome are present, atherosclerotic coronary heart disease is relatively rare when blood LDL levels are very low. In population studies, only when LDL levels begin to rise does the incidence of coronary heart disease begin to increase. Moreover, interventions which lower LDL cholesterol, including administration of HMGCoA reductase inhibitors or fibrates, reduce the prevalence of coronary heart disease. The link between blood LDL levels and insulin resistance has not been extensively studied. Clearly many factors other than insulin resistance contribute to elevated LDL. However, when there is fat overload in the liver, the production of lipoproteins by the liver appears to be increased; this overproduction of lipoproteins containing apolipoprotein B will lead to some rise in LDL levels. For example, obese persons have higher LDL-cholesterol levels than do lean persons. Thus it is not possible to remove elevated LDL entirely from the metabolic syndrome.

Other abnormalities in blood lipids are more characteristic of the metabolic syndrome. There typically are three abnormalities that group together, hence their name, the lipid triad. These include raised triglycerides, small LDL particles, and low HDL cholesterol levels. The lipid triad also has been called the atherogenic lipoprotein phenotype or atherogenic dyslipidemia. Each component of atherogenic dyslipidemia appears to independently promote atherosclerosis. Raised triglycerides indicate the presence of remnant lipoproteins, which seemingly are as atherogenic as LDL. Small LDL slip into the arterial wall more readily than normal-sized triglycerides, and thus have enhanced atherogenicity.

Low HDL probably promotes atherosclerosis in several ways. One notable example is the ability of HDL to remove excess cholesterol from the arterial wall (reverse cholesterol transport); when HDL is low, reverse cholesterol transport is retarded.

A fourth abnormality often accompanies the lipid triad. This is an elevation of apolipoprotein B (apo B). Apo B is the major lipoprotein of LDL and triglyceride-rich lipoproteins. Some investigators believe that the total apo B1' level is the single best indicator for the presence of atherogenic dyslipidemia. Certainly, when total apo B levels are high, a person is at increased risk for coronary heart disease. Patients with insulin resistance often have atherogenic dyslipidemia. When the liver is overloaded with fat, there is an overproduction of apo-B containing lipoproteins. This leads to raised triglycerides, increased remnants lipoproteins, increased total apo B, and small LDL. All of these represent a compensatory response by the liver in its attempt to cope with and remove excess fat.

In addition, an important liver enzyme, hepatic lipase, also is increased in the presence of insulin resistance. This enzyme degrades HDL and contributes to the low HDL associated with insulin resistance.

The glucocorticoid hormones (cortisol, corticosterone) produced by the adrenal gland also have the potential to cause insulin resistance. This action is observed most dramatically in patients who have Cushing's syndromes, such as Cushing's disease, which are due to overproduction of corticosteroids. Patients with Cushing's syndromes manifest insulin resistance, and many develop type 2 diabetes. Moreover, patients who receive natural or synthetic glucocorticoids in treatment of disease also show insulin resistance.

Recently a novel and important level of control of glucocorticoid action has become apparent, pre-receptor metabolism by 11β-hydroxysteroid dehydrogenases (11β-HSDs). 11β-HSDs catalyse the interconversion of active physiological 11-hydroxy glucocorticoids (cortisol in most mammals, corticosterone in rats and mice) and their inert 11-keto forms (cortisone, 11-dehydrocorticosterone). There are two isozymes of 11β-HSD, the products of distinct genes (5, 6). 11β-HSD type 2 is a high affinity dehydrogenase that rapidly inactivates corticosterone in kidney and colon, thus excluding glucocorticoids from otherwise non-selective mineralocorticoid receptors *in vivo* (7, 8). However, white adipose tissue solely expresses 11β-HSD type 1 (9), as does the liver where the enzyme is particularly abundant (10, 11).

11β-HSD-1 is a predominant reductase in most intact cells, including hepatocytes (12), adipocytes (13), neurons (14), and in the isolated liver *ex vivo* (15). This reaction direction regenerates active glucocorticoids within cells from free circulating inert 11-ketosteroids. Mice homozygous for targeted disruption of the 11βHSD-1 gene are viable, fertile and have normal longevity (16). However, 11βHSD-1 null mice cannot regenerate corticosterone from inert 11-dehydrocorticosterone, indicating this isozyme is the unique 11β-reductase. Strikingly, the null animals exhibit attenuated gluconeogenic responses upon stress and resist the hyperglycaemia induced by chronic high fat feeding (16). This occurs despite modestly elevated plasma levels of corticosterone. The results suggest that 11βHSD-1-reductase activity is an important amplifier of intrahepatic glucocorticoid action in vivo. Intriguingly, tissue-specific alterations in 11 βHSD-1 activity have been implicated in the development of obesity and insulin resistance in obese Zucker rats (4) and in humans (2; 54).

In the Metabolic Syndrome, dyslipidaemia is characterised by hypertriglyceridaemia and an aberrant lipoprotein and cholesterol profile with elevated VLDL¹, but reduced 'cardioprotective' HDL cholesterol (17). The plasma lipid profile is largely determined by gene expression in the liver. Furthermore, expression and activity of many liver proteins involved in lipid metabolism, synthesis, packaging and export are glucocorticoid-sensitive. However, the precise role of glucocorticoids in the pathogenesis of hepatic lipid metabolism is unclear, with overall effects apparently dependent upon steroid concentrations, the levels of other hormones, particularly insulin, and on diet. Indeed, many studies have used short-term treatments and/or non-physiological levels of glucocorticoids, making any extrapolations of the subtle effects of altered intracellular glucocorticoid metabolism difficult. Moreover, glucocorticoids also have important indirect effects, regulating other key transcription factors controlling lipid metabolism, notably inducing the peroxisome proliferator-activated receptor-α (PPARα) (18, 19). PPARα drives the oxidative adaptation to fasting (20, 21) and serves as the molecular target of hypolipidaemic fibrate drugs (22, 23).

The wide range of anti-inflammatory and metabolic effects of the glucocorticoids leads to their use in the treatment of a variety of diseases. The general indications for glucocorticoid therapy include ocular disease, hepatic disorders, malignant haematological disease, solid tumours, intestinal disease, and most prominently immune-mediated and inflammatory-mediated disease. However, glucocorticoid administration is associated with side-effects, which can limit the use of such therapies. Dysregulation of the lipid profile, and the metabolic syndrome, are common side-effects of glucocorticoid administration.

### Summary of the Invention

It has been determined that 11β-HSD-1^{-/-} mice have an altered cardiovascular risk profile due to liver-dependent changes in lipid metabolism and insulin sensitivity. This has been demonstrated by analysis of circulating lipids and lipoproteins and the expression of hepatic genes involved in lipid metabolism and transport, as well as fibrinogen, another glucocorticoid-sensitive hepatic transcript associated with cardiovascular risk. The findings reported herein demonstrate that a reduction in 11β-HSD1 leads to an atheroprotective lipid profile which counteracts the effects of insulin resistance and metabolic syndrome.

According to a first aspect, therefore, the invention provides for the combined use of an agent which reduces intracellular 11β-HSD1 activity and a PPARα agonist in the manufacture of a composition for the promotion of an antheroprotective lipid profile.

As set forth above, reduced levels of HDL and increase levels of plasma triglycerides, the major component of LDL, are major contributors to cardiovascular risk and atherosclerosis. In accordance with the present invention, it is provided that inhibition of 11β-HSD1 leads to reduction in plasma triglycerides and thus LDL, and an increase in HDL. The lipid profile of individuals at risk from coronary heart disease, or other cardiovascular complaints, especially those linked with suboptimal cholesterol metabolism, may be improved by reduction of 11 β-HSD1 levels.

Agents which reduce intracellular 11β-HSD1 activity include those agents which modify the genetic profile of an individual in order to downregulate 11β-HSD1 gene expression. Thus, the invention encompasses approaches involving gene therapy to delete or downregulate endogenous 11β-HSD1 genes. Such approaches include antisense nucleic acid approaches, which are capable of reducing or preventing the transcription and/or translation of mRNA in vivo, and other methods for genetic manipulation which act at the mRNA level; and genetic manipulation of endogenous genes to reduce levels of their expression in somatic tissues.

Preferably, the agent which lowers 11β-HSD1 levels is an inhibitor of 11β-HSD1 synthesis or activity. Thus, as set out below, "levels" should be understood to refer to the activity of 11β-HSD1 and not necessarily to the physical amounts of this enzyme present in tissues or cells. Inhibitors of 11β-HSD 1 are known in the art, and further described below.

Advantageously, the lipid profile of the treated individual shows a reduction in plasma triglycerides and/or an increase in HDL cholesterol. Such lipid profiles are acknowledged to be atheroprotective.

PPARα promotes fatty acid oxidation in the liver; as shown below, inhibition of 11β-HSD1 leads to upregulation of PPARα, which in turn leads to reduction in plasma triglycerides. Inhibition of 11βHSD-1 also increases the expression of PPARγ in adipose tissue, which has beneficial effects on insulin sensitivity, lipid profile, glucose tolerance and cardiovascular risk.

The PPARα agonist may for example be a fibrate. Fibrates activate PPARα, lower plasma triglycerides and repress apoCIII; a combination therapy comprising both a fibrate and an agent which reduces 11β-HSD1 activity confers a highly favourable lipid profile.

Preferably, the treated individual moreover shows a reduction in plasma fibrinogen. Fibrinogen is an acknowledged independent risk factor for cardiovascular disease.

Advantageously, the invention also provides for a reduction in serum apoCIII levels. ApoCIII increases plasma triglyceride levels by inhibiting hepatic glycolysis. As described herein, inhibition of 11β-HSD1 activity leads to a reduction in serum apoCIII. Accordingly, the invention provides for the use of an agent which reduces intracellular 11β-HSD1 activity and a PPARα agonist in the production of a composition for the reduction of apoCIII levels in an individual. ApoCIII is known to be positively correlated with cardiovascular disease risk.

The 11 β-HSD I activity is preferably an intracellular 11β-HSD1 activity.

There is described herein the use of an agent which reduces intracellular 11 β-HSD 1 activity in the production of a composition for the promotion of insulin sensitivity. As set out above, insulin resistance is associated with cardiovascular risk and the metabolic syndrome. Reduction of 11β-HSD1 levels leads to an increase in insulin sensitivity.

There is also described herein the use of an agent which reduces intracellular 11β-HSD1 activity in the production of a composition for the improvement of glucose tolerance in an individual. Reduction in 11β-HSD1 levels lead to improved dynamic glycaemic control. This is in keeping with the effects observed in improvements in insulin sensitivity.

In a further embodiment, the invention provides a pharmaceutical composition comprising a PPARα agonist and an agent which reduces 11 β-HSD1 activity. The pharmaceutical composition according to the invention may be provided as a combined preparation, or as a kit comprising both a PPARα agonist and an agent which reduces 11β-HSD1 activity for simultaneous, simultaneous separate or sequential use.

There is provided herein a method for reducing cardiovascular disease risk in a subject at risk of cardiovascular disease, comprising administering to said subject a pharmaceutically effective amount of an agent which reduces 11β-HSD1 activity in combination with a PPARγ agonist.

In a still further embodiment, the invention provides a pharmaceutical composition comprising a PPARγ agonist and an agent which reduces 11β-HSD1 activity. The pharmaceutical composition according to the invention may be provided as a combined preparation, or as a kit comprising both a PPARγ agonist and an agent which reduces 11β-HSD1 activity for simultaneous, simultaneous separate or sequential use.

Methods, uses and compositions according to the invention are useful in the treatment of a variety of conditions which are associated with increased risk of cardiovascular disease.

### Brief Description of the Figures

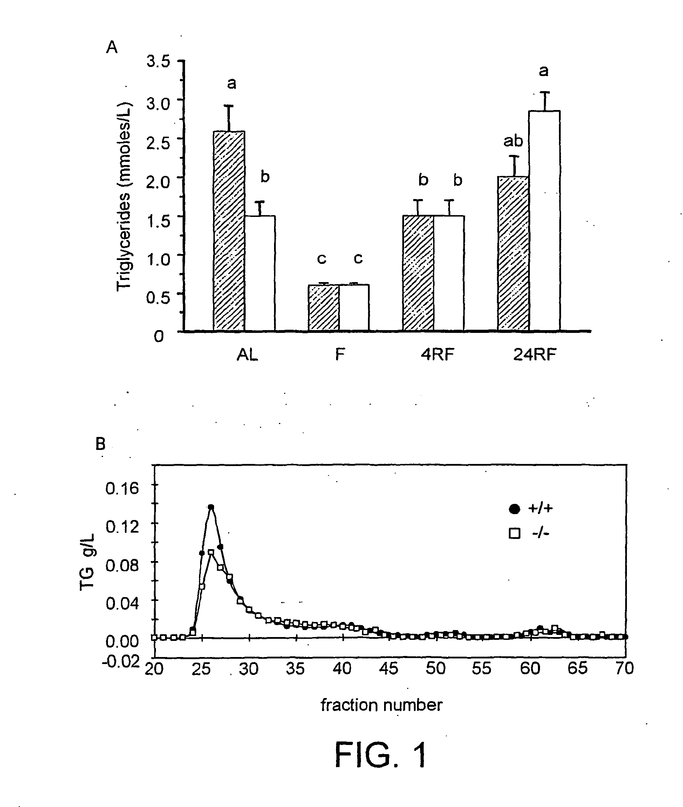

**Figure 1****. *A.*** Triglyceride levels in wild type (solid bars) versus 11βHSD-1^{-/-} (open bars) animals subjected to dietary manipulation: **AL;** ad lib fed, **F;** 24h fasted, **4RF;** 24h fast with 4h re-feed and **24RF;** 24h fast with 24h re-feed. Lower case letter superscripts identify groups that are similar statistically. ***B.*** Representative true triglyceride FPLC profile from *ad lib* fed wild type and 11βHSD-1^{*-*/*-*} mice. Note the lower ad lib fed triglyceride levels in 11β-HSD1 ^{-/-} mice.

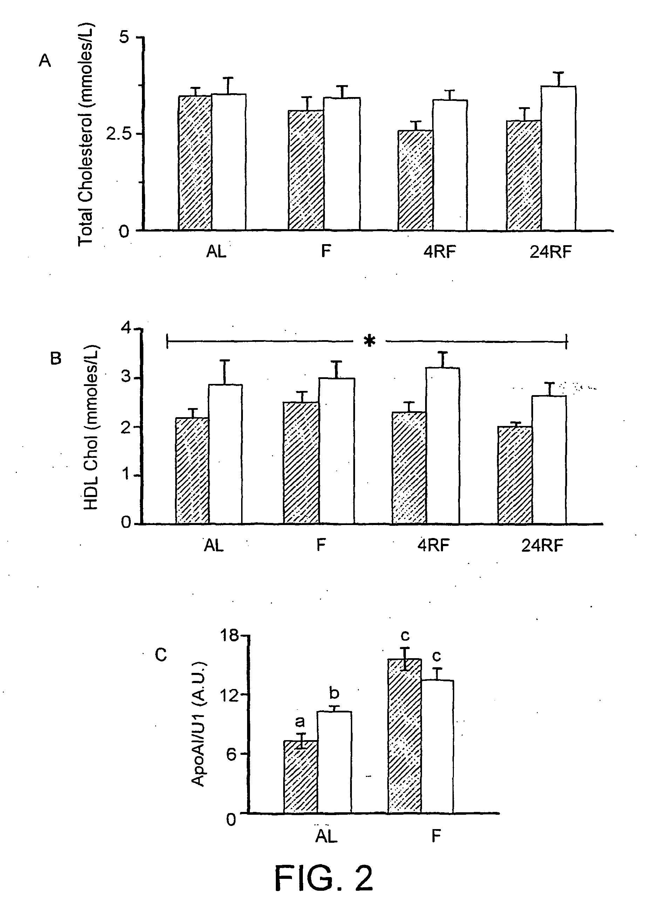

**Figure 2****. A.** Total cholesterol levels in wild type (solid bars) versus 11βHSD-1^{-/-} (open bars) animals subjected to dietary manipulation: **AL;** ad lib fed, **F;** 24h fasted, **4RF;** 24h fast with 4h re-feed and **24RF;** 24h fast with 24h re-feed. ***B*.** HDL cholesterol levels in wild type (solid bars) versus 11βHSD-1^{-/-} (open bars) animals subjected to dietary manipulation: **AL;** ad lib fed, **F;** 24h fasted, **4RF;** 24h fast with 4h re-feed and **24RF;** 24h fast with 24h re-feed. (* significantly greater values in 11β-HSD1^{-/-} mice than wild type). C. Hepatic apolipoprotein AI mRNA levels (encoding the major component of the HDL particle) in wild type (solid bars) versus 11βHSD-1^{-/-} (open bars) animals. Lower case letter superscripts identify groups that are similar statistically. Note the significantly higher HDL cholesterol levels in 11β-HSD1 -/- mice, as well as higher fed apolipoprotein AI mRNA in fed 11β-HSD-1^{-/-} mouse liver.

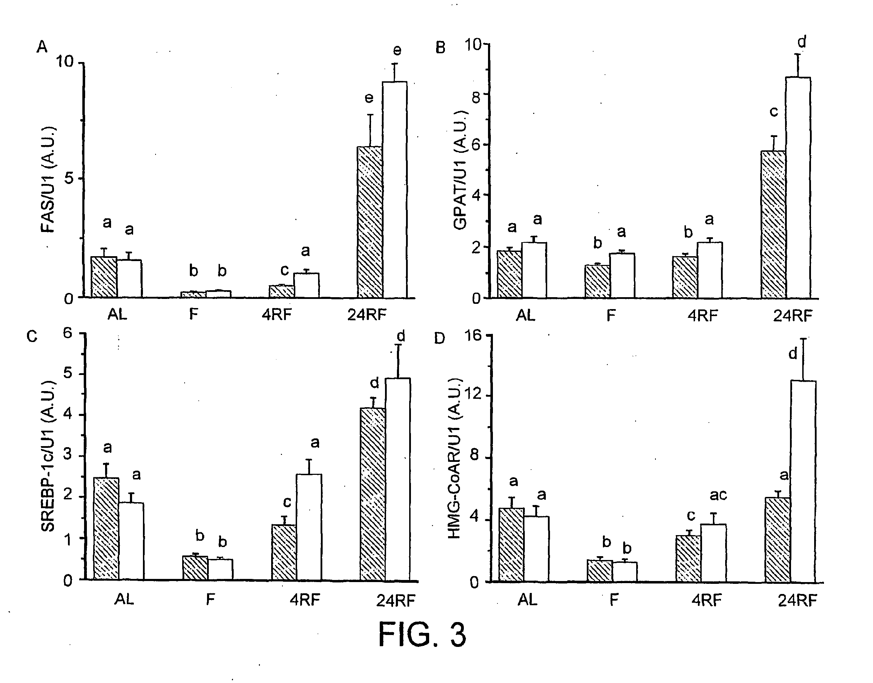

**Figure 3****.** Transcript levels of proteins of the lipogenic (*A-C*) and cholesterol biosynthesis pathways (D) in livers of wild type (solid bars) versus 11βHSD-1^{-/-} (open bars) animals subjected to dietary manipulation: **AL;** ad lib fed, **F;** 24h fasted, **4RF;** 24h fast with 4h re-feed and **24RF;** 24h fast with 24h re-feed. Transcript levels were analysed by northern blot as described in Materials and Methods. ***A*.** Fatty acid synthase transcript levels. ***B*.** Glycerolphosphate acyl transferase transcript levels. ***C*.** Sterol regulatory element binding protein-1c transcript levels. ***D.*** Hydroxy-methyl-glutaryl CoA synthase transcript levels. Transcript levels were corrected for RNA loading by using a cDNA probe for the U1 small ribonucleoprotein. Lower case letter superscripts identify groups that are similar statistically. Note the unaltered levels of key enzymes of triglyceride and cholesterol biosynthesis in 11β-HSD1^{-/-} mice suggesting that this does not account for the reduced triglycerides seen. Upon re-feeding SREBP-1c, FAS, GPAT and HMG-CoAR, were more rapidly and/or markedly induced in 11β-HSD-1^{-/-} mice, implying 11β-HSD-1^{-/-} liver has greater insulin action or sensitivity in terms of lipogenic pathways.

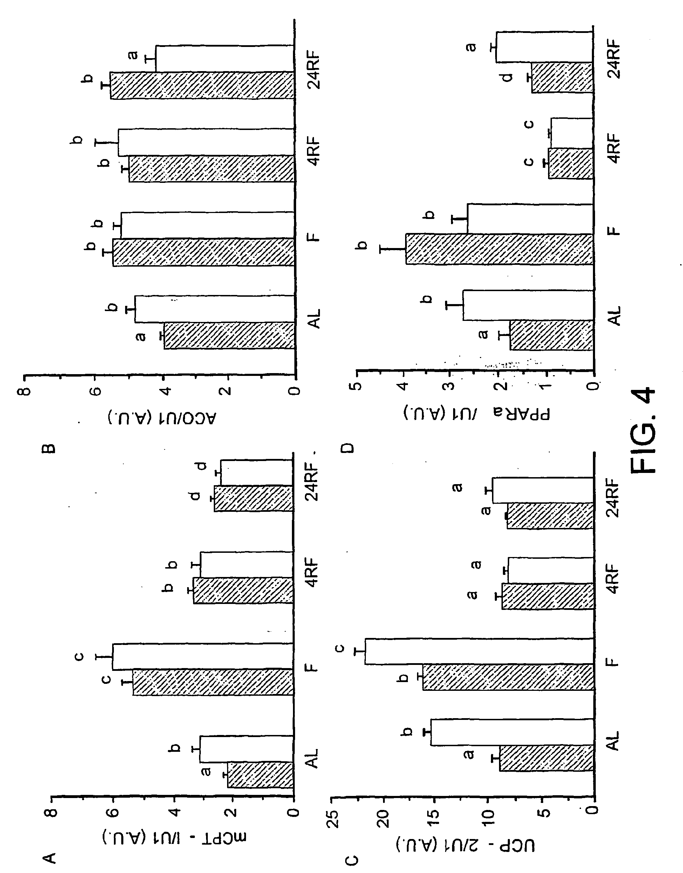

**Figure 4****.** Transcript levels of proteins in the fatty acid oxidation pathway in livers of wild type (solid bars) versus 11βHSD-1^{-/-} (open bars) animals subjected to dietary manipulation: **AL;** ad lib fed, F; 24h fasted, **4RF;** 24h fast with 4h re-feed and **24RF;** 24h fast with 24h re-feed. Transcript levels were analysed by northern blot as described in Materials and Methods. ***A*.** Carnitinepalmitoyltransferase-I (CPT-I) transcript levels. *B*. Acyl coA oxidase transcript levels. ***C*.** Uncoupling protein-2 transcript levels. ***D*.** Peroxisome proliferator-activated receptor-α transcript levels. Transcript levels were corrected for RNA loading by using a cDNA probe for the U1 small ribonucleoprotein. Lower case letter superscripts identify groups that are similar statistically. Note the increased expression of key enzymes of β-oxidation and their driving transcription factor PPARalpha in ad lib fed 11β-HSD1-/- mice. The data suggest that enhanced triglyceride metabolism underlies the reduction in plasma levels seen in 11β-HSD1-/- mice.

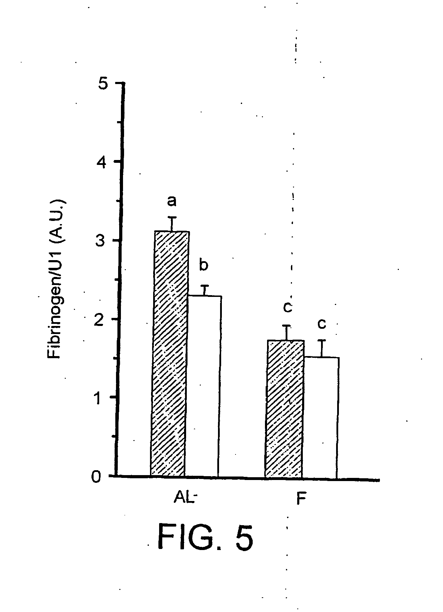

**Figure 5****.** Hepatic Aα-fibrinogen mRNA expression in 11β-HSD-1-/- mice. Transcript levels Aα-fibrinogen in livers of wild type (solid bars) versus 11β-HSD-1^{-/-} (open bars) animals that are: **AL;** *ad lib* fed, **F;** 24h fasted. Lower case letter superscripts identify groups that are similar statistically. Transcript levels were corrected for RNA loading by using a cDNA probe for the U1 small ribonucleoprotein. Note that the independent cardiovascular risk factor, Aα-fibrinogen, transcript levels are reduced in 11β-HSD1-/mice, further indicating a 'cardioprotective' phenotype.

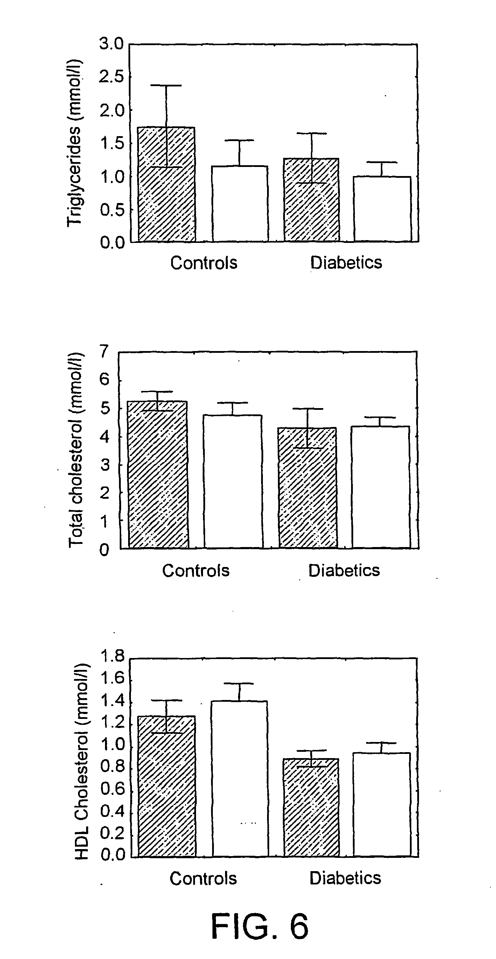

**Figure 6****.** Effect of administration of carbenoxolone on fasting plasma lipids in healthy humans and patients with type 2 diabetes mellitus. 6 men with type 2 diabetes mellitus and 6 healthy controls were administered placebo (filled bars) and carbenoxolone (open bars) in a randomised double-blind crossover study, as known in the art. Fasting levels of plasma lipids are shown.

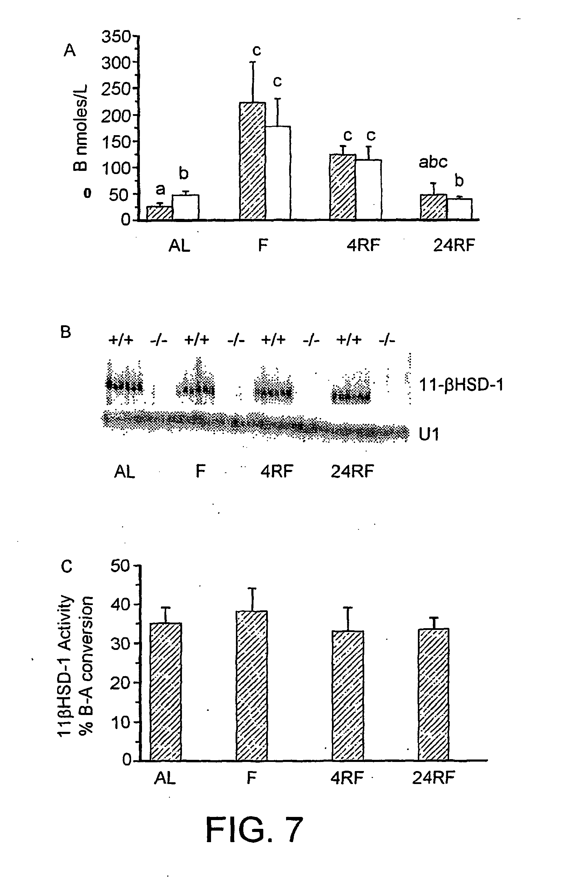

**Figure 7****.** The effects of feeding status on plasma corticosterone levels in 11β-HSD-1^{-/-}mice and on wild type 11β-HSD-1 mRNA and activity. ***A.*** Corticosterone levels, ***B.*** 11β-HSD-1 mRNA and ***C.*** 11βHSD-1 activity (percentage conversion of corticosterone to 11-dehydrocorticosterone as outlined in Experimental Procedures) in wild type (solid bars) versus 11β-HSD-1^{-/-} (open bars) animals that are: **AL;** *ad lib* fed, **F;** 24h fasted, **4RF;** 24h fasted with a 4h re-feed and **24RF;** 24h fasted with a 24h re-feed.

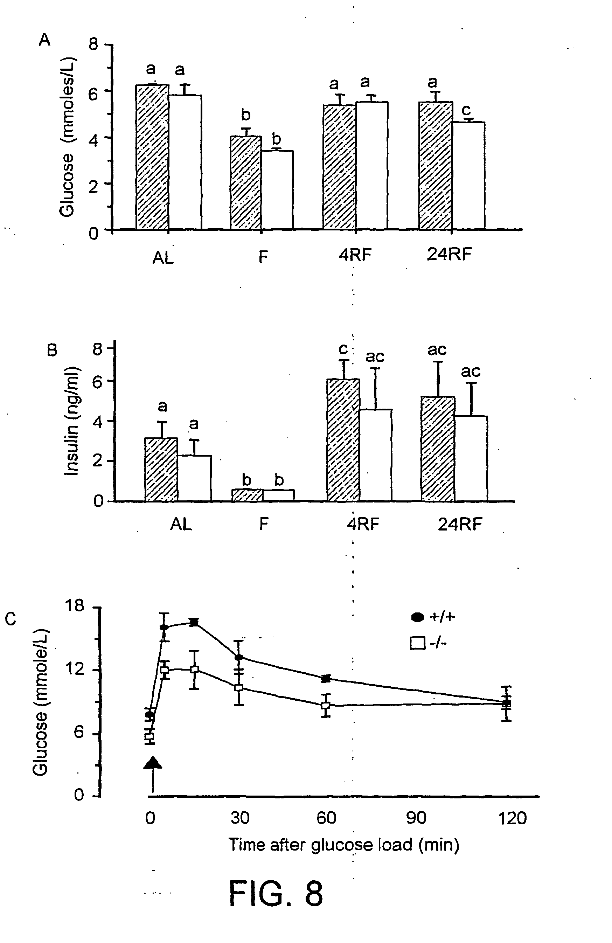

**Figure 8****.** The effects of dietary status and 11β-HSD-1 knockout and plasma glucose, insulin and dynamic glucose disposal after intraperitoneal glucose administration. A. Plasma glucose and ***B.*** plasma insulin in wild type (solid bars) versus 11β-HSD-1^{-/-} (open bars) animals that are: **AL;** *ad lib* fed, **F;** 24h fasted, **4RF;** 24h fasted with a 4h re-feed and **24RF;** 24h fasted with a 24h re-feed. ***C.*** Dynamics of glucose disposal upon intraperitoneal glucose load (2mg/g body weight) following a 16 hour fast in wild type (filled circle) and 11β-HSD-1^{-/-} mice (empty box).

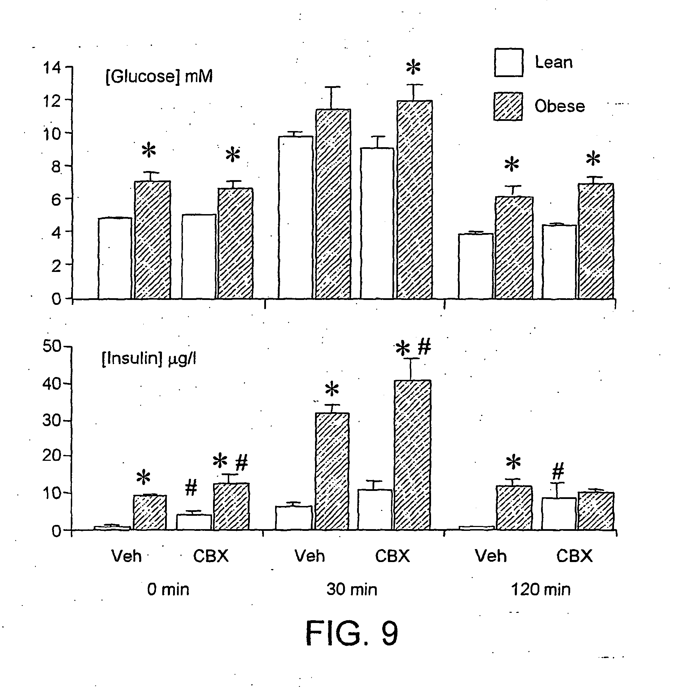

**Figure 9****.** Effect of carbenoxolone administration on glucose tolerance in lean and obese Zucker rats. Veh denotes vehicle treated animals and CBX denotes carbenoxolone treated animals. Data are mean ± SEM; * denotes p<0.05 comparing lean and obese animals in the same treatment group; # denotes p<0.05 compared with vehicle treated group of the same phenotype.

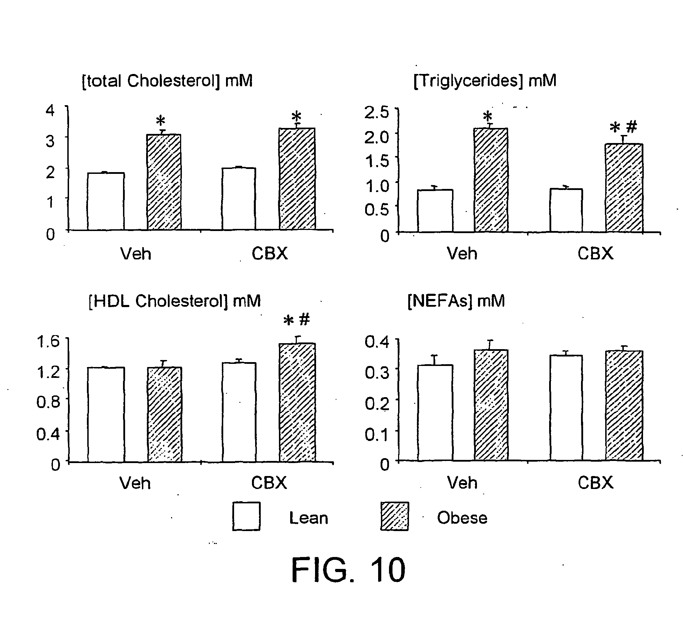

**Figure 10****.** Effect of carbenoxolone administration on plasma lipid profile in lean and obese Zucker rats. Veh denotes vehicle treated animals and CBX denotes carbenoxolone treated animals. NEFAs are non-esterified fatty acids. Data are mean ± SEM; * denotes p<0.05 comparing lean and obese animals in the same treatment group; # denotes p<0.05 compared with vehicle treated group of the same phenotype.

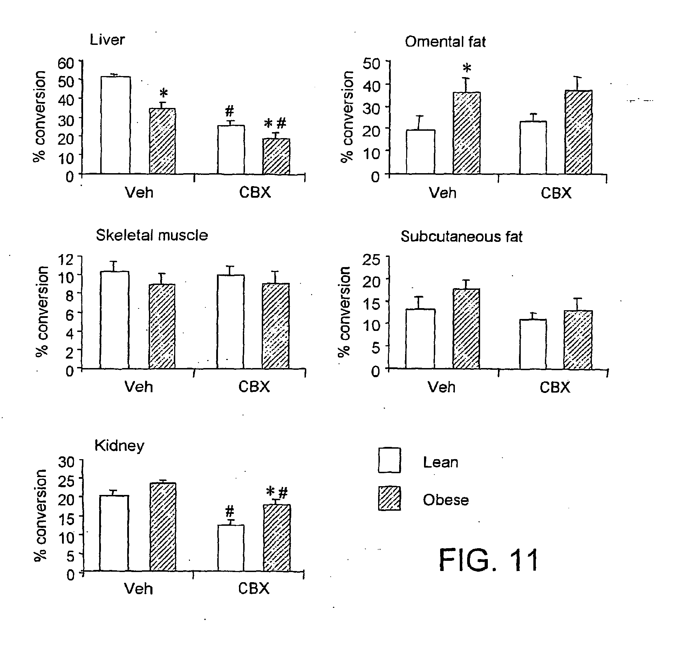

**Figure 11****.** Effect of carbenoxolone administration on 11β-HSD1 activity in different tissues in lean and obese Zucker rats. Veh denotes vehicle treated animals and CBX denotes carbenoxolone treated animals. 11β-HSD activity is expressed as percent conversion of corticosterone to 11-dehydrocorticosterone. Data are mean ± SEM; * denotes p<0.05 comparing lean and obese animals in the same treatment group; # denotes p<0.05 compared with vehicle treated group of the same phenotype.

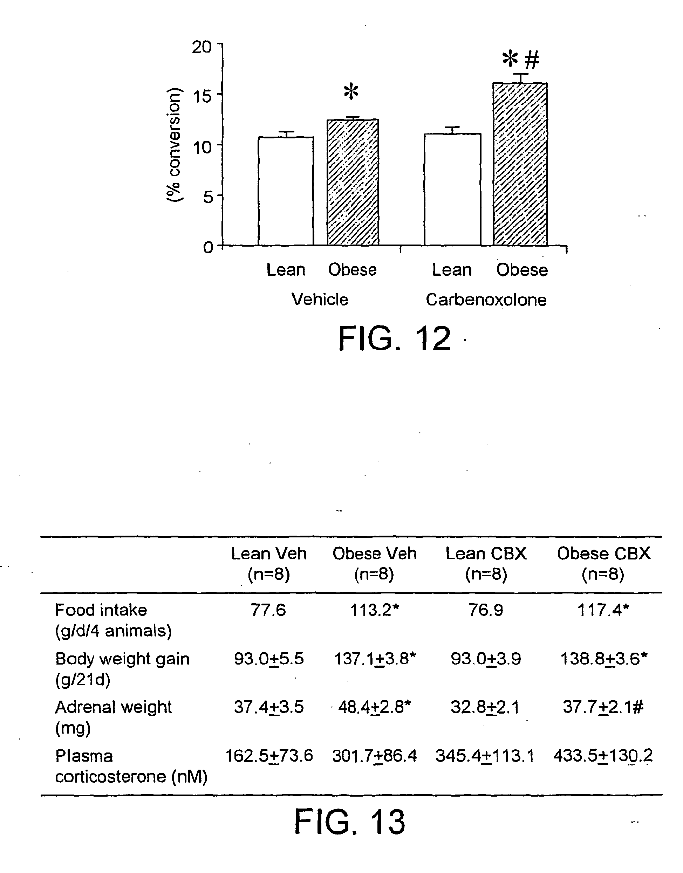

**Figure 12****.** Effect of carbenoxolone administration on hepatic 5β-reductase activity in lean and obese Zucker rats. Data are mean ± SEM; * denotes p<0.05 comparing lean and obese animals in the same treatment group; # denotes p<0.05 compared with vehicle treated group of the same phenotype. 5β-Reductase activity is expressed as percent conversion of corticosterone to 5β-tetrahydrocorticosterone.
**Figure 13**. Effect of carbenoxolone administration on metabolic and hypothalamic-pituitary-adrenal axis characteristics in lean and obese Zucker rats. Veh denotes vehicle treated animals and CBX denotes carbenoxolone treated animals. Data are mean ± SEM; * denotes p<0.05 comparing lean and obese animals in the same treatment group; # denotes p<0.05 compared with vehicle treated group of the same phenotype.

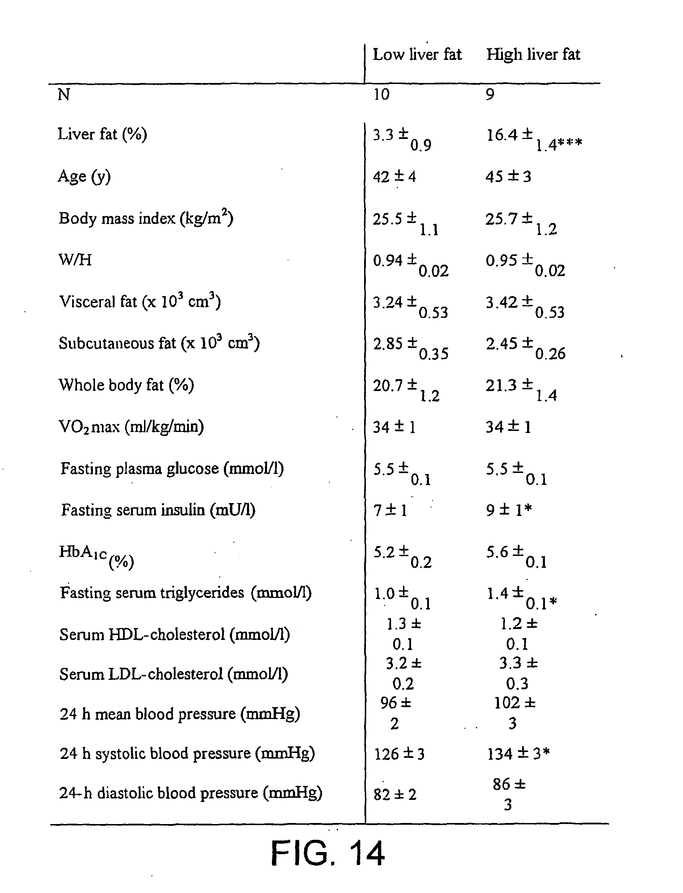

**Figure 14****.** Body composition and basal biochemistry in groups of men with contrasting liver fat content. *p<0.05 between groups, ***p<0.001.

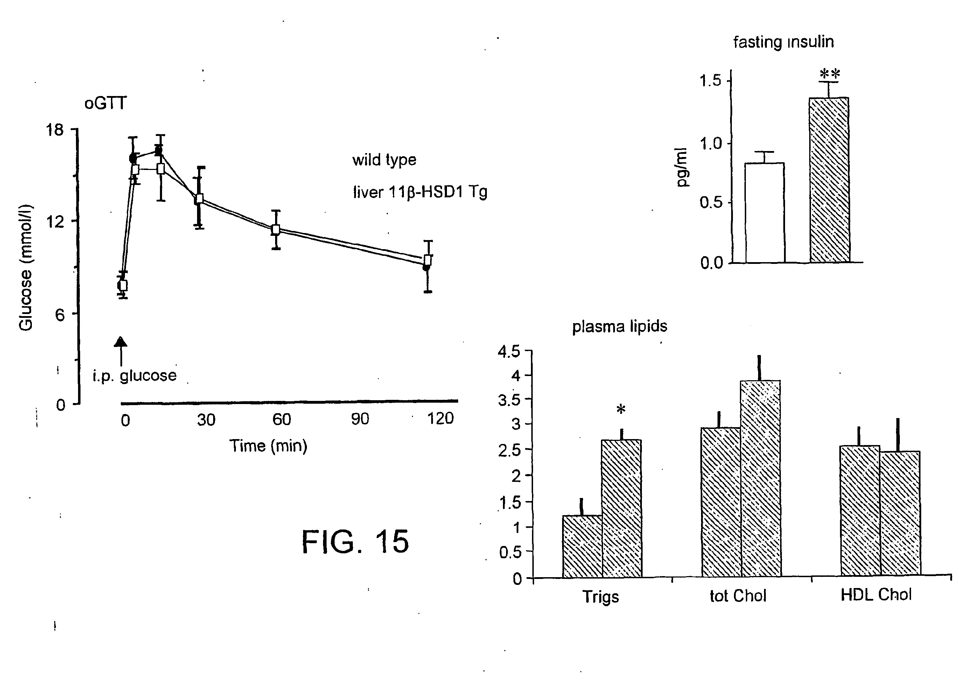

**Figure 15****.** Metabolic phenotype of mice with transgenic overexpression of 11β-HSD1 in liver under the ApoE promoter. Panels show data as mean +/- SEM from 3-6 wild type (light grey symbols) and overexpressing mice (dark grey symbols). First panel shows plasma glucose after intra-peritoneal glucose load. Second panel shows fasting plasma insulin, which was higher in overexpressor mice (** p<0.41). Third panel shows plasma lipids, with higher fasting triglycerides (p<0.05) and total cholesterol (p=0.06) in overexpressors.

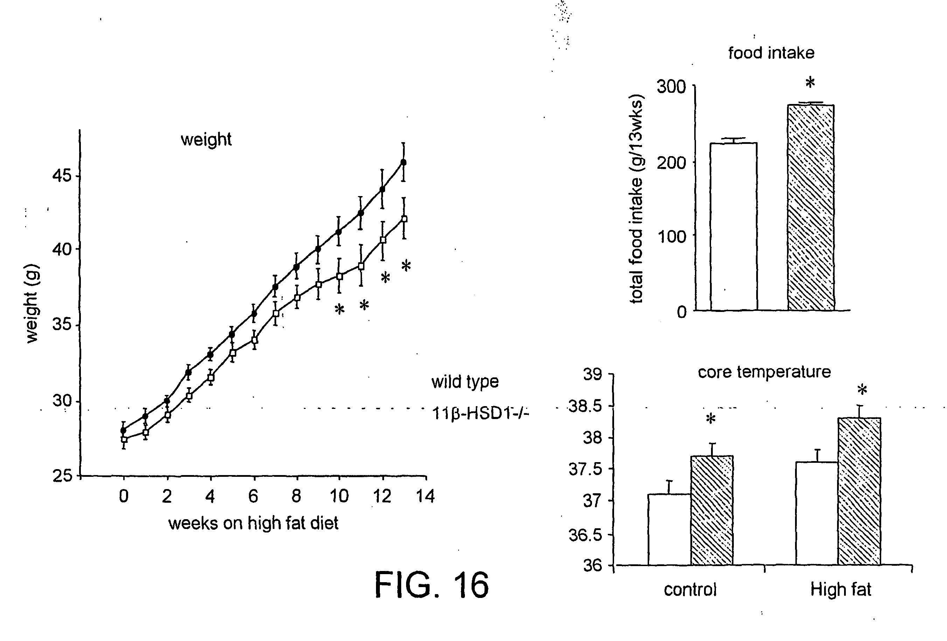

**Figure 16****.** Weight gain, food intake, and rectal temperature in 11β-HSD1 -/- and wild type mice during prolonged high fat feeding. Data are mean +/- SEM from n=7-8 mice per group for wild type (light grey symbols; circles) and 11β-HSD1 -/- (dark grey symbols; squares). First panel shows prevention of weight gain and obesity in 11β-HSD1 -/- mice (*p<0.05 vs wild type). Second panel shows initial increase, rather than decrease, in food consumption in 11β-HSD1 -/- mice (*p<0.05). Third panel shows higher rectal temperature (in degrees Centigrade) in 11β-HSD1 -/- mice both in control and high fat fed conditions (*p<0.05).

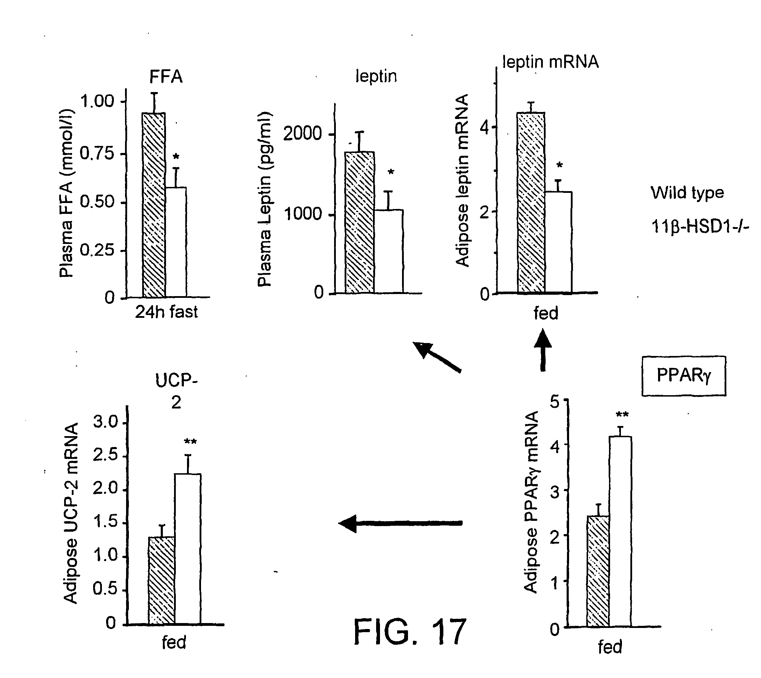

**Figure 17****.** Plasma free fatty acids (FFA) and leptin and subcutaneous adipose mRNA for leptin, uncoupling protein 2 (UCP2) and PPARgamma in 11β-HSD1 -/- mice (black symbols) and wild type controls (grey symbols). Data are mean +/- SEM for 6-8 mice per group. * indicates 0.05 for differences between groups.

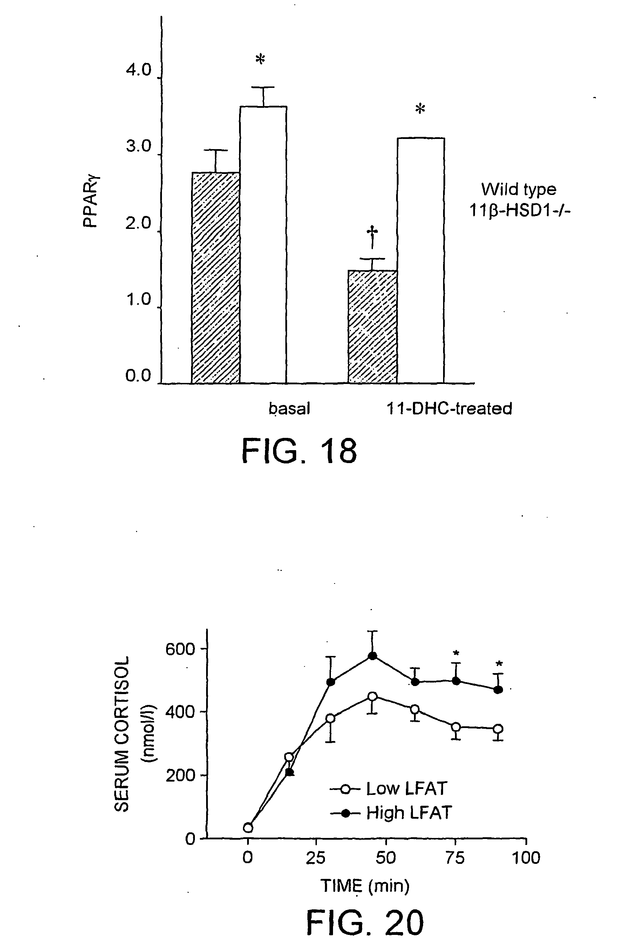

**Figure 18**. 11HSD1 -/- mice have up-regulation of PPARγ mRNA in macrophages. PPARγ is important in mediating macrophage uptake and export of oxidised or acetylated LDL-cholesterol, notably in the atheromatous plaque in blood vessel walls. The dominant effect appears to be on cholesterol export via ABCA1 and related transporters. The data imply that 11β-HSD1 inhibition would facilitate cholesterol export from macrophages in the plaque, reducing foam cell formation and their contribution to atherogenesis. Data are mean +/- SEM for n=6 mice per group for wild type controls (black symbols) and 11β-HSD1 -/- mice (grey symbols). *p<0.05 between wild type and knockout mice. 11-DHC =11-dehydrocorticosterone, which down-regulates PPARγ in wild type controls (p<0.05) but not in 11β-HSD1 -/- mice in which 11-DHC cannot be reactivated to active corticosterone.

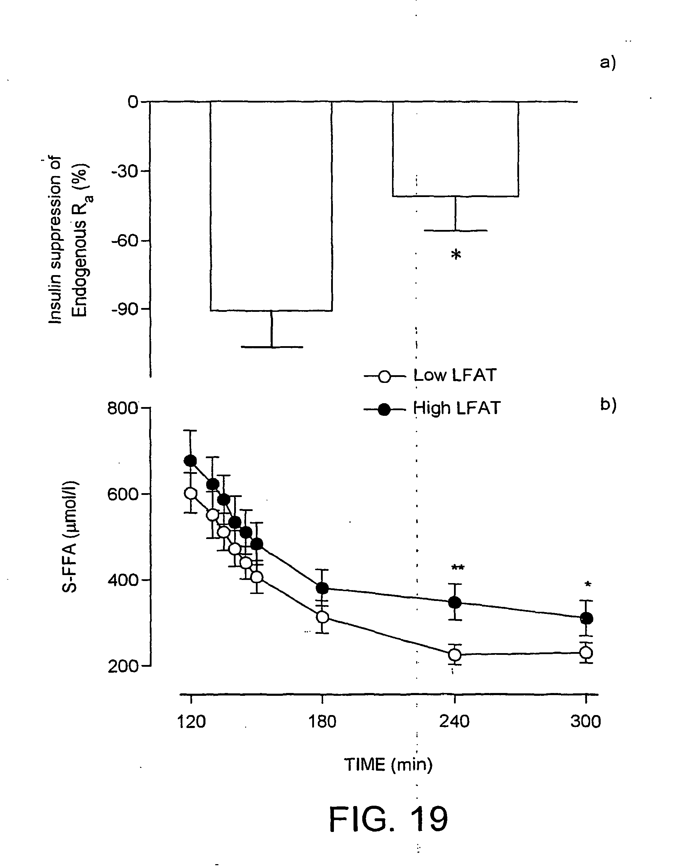

**Figure 19****.** Hepatic and peripheral insulin resistance in men with high liver fat content. Endogenous glucose Rₐ rate of appearance during the last hour of hyperinsulinemia (240-300 min) in men with low and high liver fat content (LFAT). *p<0.05 for low vs. high LFAT. b) Serum FFA concentrations at baseline before start of the insulin infusion (120 min) and during the insulin infusion (120-300 min) in men with low and high LFAT. *p<0.05, **p<0.02 for men with low vs. high LFAT.
**Figure 20****.** Increased hepatic 11β-HSD1 activity in liver of men with high liver fat content (LFAT). Serum cortisol concentrations during suppression of endogenous cortisol secretion by dexamethasone and after oral cortisone acetate in men with high vs. low LFAT. *p<0.05 for high vs. low LFAT.

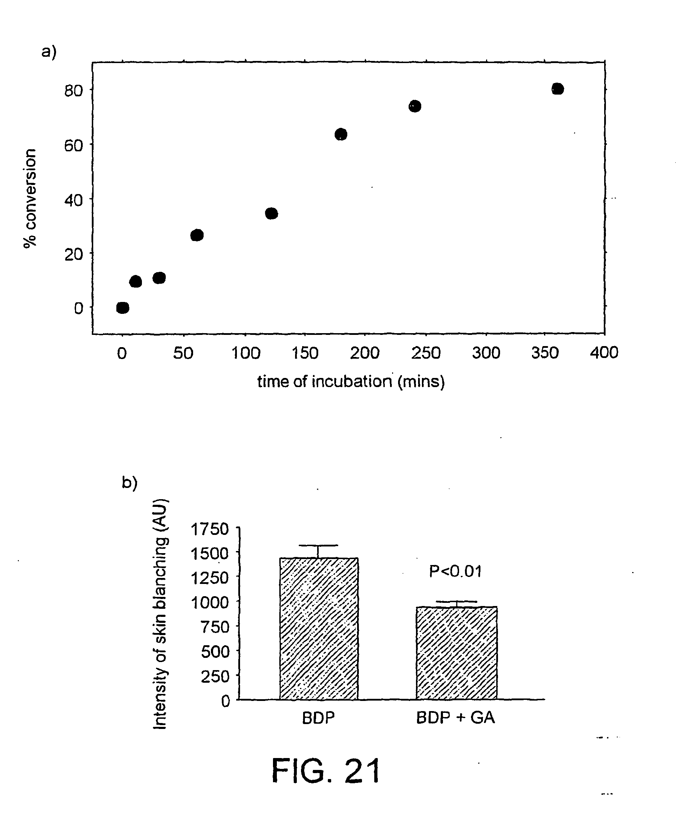

**Figure 21**. 11β-HSD1 inhibition protects against glucocorticoid effects of exogenous synthetic glucocorticoids. a) Beclomethasone incubated with homogenised rat liver is converted to 11-dehydrobeclomethasone, indicating the steroid is a substrate for 11β-HSD1. b) Topical application of beclomethasone (BDP) or BDP with the addition of the 11 β-HSD inhibitor glycyrrhetinic acid (GA) induces skin blanching in healthy human skin in vivo, measured here as the area under the dose-response curve in arbitrary units. GA attenuates the effect of beclomethasone in this bioassay.

### Detailed Description of the Invention

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. and chemical methods.

### Definitions

An "agent", as used herein, is any substance which has the desired effect on 11β-HSD1 activity. Thus, the agent may be a chemical compound, such as a small molecule or complex organic compound, a protein, an antibody or a genetic construct which acts at the DNA or mRNA level in an organism. The agent may act directly or indirectly, and may modulate the activity of a substance which itself modulates the activity of 11β-HSD1.

A "lipid profile" is the level of lipids present in the blood. A lipid profile usually includes the total cholesterol, high density lipoprotein (HDL) cholesterol, triglycerides, and the calculated low density lipoprotein (LDL) cholesterol. In the present invention, a lipid profile comprises at least the level of one or more triglycerides and the level of HDL cholesterol.

An "atheroprotective" profile is a profile which prevents, offsets or ameliorates the pathogenesis of atherosclerosis.

"Expression", as in gene expression, is used herein to refer to the process of transcription and translation of a gene to produce a gene product, be it RNA or protein. Thus, inhibition of expression may occur at any one or more of many levels, including transcription, post-transcriptional processing, translation, post-translational modification, and the like. Agents which modulate gene expression, including transcription or translation, include for example agents which downregulate or knock out endogenous genes; including agents which knock out genes in pluripotent cells which give rise to all or part of an animal.

Inhibition of 11β-HSD 1 "synthesis or activity" refers to the inhibition of 11β-HSD1 at the protein level, to prevent or downregulate the production of the protein, or at least one biological activity of the protein once produced.

"Cardiovascular disease risk" is the risk, as measured according to accepted risk factors, to which an animal is exposed of suffering from one or more cardiovascular complaints or pathologies. Cardiovascular disease (CVD) includes coronary heart disease (CHD) and stroke. The measurement of risk itself is largely statistical; in the context of the present invention, the presence or absence of factors which are accepted to contribute to increasing or decreasing the risk of CVD according to statistical analyses are taken as indicative of increased or decreased risk respectively.

A pharmaceutically effective amount is an amount of a composition which achieves the desired effect in an animal. The actual amount will vary on a number of factors, as known to those skilled in the art. Using the guidance given herein and knowledge of the art, the determination of a pharmaceutically effective amount is within the ordinary skill of a physician. Pharmaceutically effective amounts designed for particular applications may be packaged as unit doses to facilitate administration.

### 11-β Hydroxysteroid Dehydrogenase Type 1

11β-HSD1 is known in the art (A. K. Agarwal, C. Monder, B. Eckstein, and P. C. White. Cloning and expression of rat cDNA encoding corticosteroid 11β-dehydrogenase. J.Biol.Chem. 264:18939-18943, 1989) and is commonly expressed in white adipose tissue and liver. The structure of 11β-HSD1 and the human gene encoding it are known (GenBank NM_005525.1 GI:5031764). Human cDNA clones encoding 11β-hydroxysteroid dehydrogenase type I were isolated from a testis cDNA library by hybridisation with the previously isolated rat 11-HSD cDNA clone (Tannin, et al., J. Biol. Chem. 266: 16653-16658, 1991). The cDNA contained an open reading frame of 876 nucleotides, which predicted a protein of 292 amino acids. The sequence was 77% identical at the amino acid level to the rat 11-HSD. By hybridisation of the human cDNA to a human/hamster hybrid cell panel(72) localised the 11β-HSD1 gene to chromosome 1. The localisation was confirmed by isolating the gene from a chromosome 1-specific library using the cDNA as a probe. The gene consists of 6 exons and is at least 9 kb long.

### Agents which modulate 11β-HSD1 expression

The modulation of gene expression is known to those skilled in the art to be achievable in a number of ways in vivo and *in vitro.* Antisense techniques as well as direct gene manipulation are known for use in modulating gene expression. The invention thus includes the use of antisense nucleic acids, which may incorporate natural or modified nucleotides, or both, ribozymes, including hammerhead ribozymes, gene knockout such as by homologous recombination, and other techniques for reducing gene expression levels.

Nucleic acid agents may be produced and expressed according to techniques known in the art. Nucleic acids encoding desired agents can be incorporated into vectors for manipulation and expression. As used herein, vector (or plasmid) refers to discrete elements that are used to introduce heterologous DNA into cells for either expression or replication thereof. Selection and use of such vehicles are well within the skill of the artisan. Many vectors are available, and selection of appropriate vector will depend on the intended use of the vector, i.e. whether it is to be used for DNA amplification or for DNA expression, the size of the DNA to be inserted into the vector, and the host cell to be transformed with the vector. Each vector contains various components depending on its function (amplification of DNA or expression of DNA) and the host cell for which it is compatible. The vector components generally include, but are not limited to, one or more of the following: an origin of replication, one or more marker genes, an enhancer element, a promoter, a transcription termination sequence and a signal sequence.

Both expression and cloning vectors generally contain nucleic acid sequence that enable the vector to replicate in one or more selected host cells. Typically in cloning vectors, this sequence is one that enables the vector to replicate independently of the host chromosomal DNA, and includes origins of replication or autonomously replicating sequences. Such sequences are well known for a variety of bacteria, yeast and viruses. The origin of replication from the plasmid pBR322 is suitable for most Gram-negative bacteria, the 2m plasmid origin is suitable for yeast, and various viral origins (e.g. SV 40, polyoma, adenovirus) are useful for cloning vectors in mammalian cells. Generally, the origin of replication component is not needed for mammalian expression vectors unless these are used in mammalian cells competent for high level DNA replication, such as COS cells.

Most expression vectors are shuttle vectors, i.e. they are capable of replication in at least one class of organisms but can be transfected into another class of organisms for expression. For example, a vector is cloned in E. coli and then the same vector is transfected into yeast or mammalian cells even though it is not capable of replicating independently of the host cell chromosome. DNA may also be replicated by insertion into the host genome.

Advantageously, an expression and cloning vector may contain a selection gene also referred to as selectable marker. This gene encodes a protein necessary for the survival or growth of transformed host cells grown in a selective culture medium. Host cells not transformed with the vector containing the selection gene will not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available from complex media.

As to a selective gene marker appropriate for yeast, any marker gene can be used which facilitates the selection for transformants due to the phenotypic expression of the marker gene. Suitable markers for yeast are, for example, those conferring resistance to antibiotics G418, hygromycin or bleomycin, or provide for prototrophy in an auxotrophic yeast mutant, for example the URA3, LEU2, LYS2, TRP1, or HIS3 gene.

Since the replication of vectors is conveniently done in E. coli, an E. coli genetic marker and an E. coli origin of replication are advantageously included. These can be obtained from E. coli plasmids, such as pBR322, Bluescript© vector or a pUC plasmid, e.g. pUC 18 or pUC19, which contain both E. coli replication origin and E. coli genetic marker conferring resistance to antibiotics, such as ampicillin.

Suitable selectable markers for mammalian cells are those that enable the identification of cells which have been transformed with the nucleic acid in question, such as dihydrofolate reductase (DHFR, methotrexate resistance), thymidine kinase, or genes conferring resistance to G418 or hygromycin. The mammalian cell transformants are placed under selection pressure which only those transformants which have taken up and are expressing the marker are uniquely adapted to survive. In the case of a DHFR or glutamine synthase (GS) marker, selection pressure can be imposed by culturing the transformants under conditions in which the pressure is progressively increased, thereby leading to amplification (at its chromosomal integration site) of both the selection gene and the linked DNA.

Expression and cloning vectors usually contain a promoter that is recognised by the host organism and is operably linked to the desired nucleic acid. Such a promoter may be inducible or constitutive. The promoters may be operably linked to the nucleic acid in question by removing the promoter from the source DNA, for example by restriction enzyme digestion, and inserting the isolated promoter sequence into the vector. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Their nucleotide sequences have been published, thereby enabling the skilled worker operably to ligate them into vectors as required, using linkers or adaptors to supply any required restriction sites. Promoters for use in bacterial systems will also generally contain a Shine-Delgarno sequence.

Preferred expression vectors are bacterial expression vectors which comprise a promoter of a bacteriophage such as phagex or T7 which is capable of functioning in the bacteria. In one of the most widely used expression systems, the nucleic acid encoding the fusion protein may be transcribed from the vector by T7 RNA polymerase (73). In the *E. coli* BL21(DE3) host strain, used in conjunction with pET vectors, the T7 RNA polymerase is produced from the λ-lysogen DE3 in the host bacterium, and its expression is under the control of the IPTG inducible lac UV5 promoter. This system has been employed successfully for over-production of many proteins. Alternatively the polymerase gene may be introduced on a lambda phage by infection with an int- phage such as the CE6 phage which is commercially available (Novagen, Madison, USA). other vectors include vectors containing the lambda PL promoter such as PLEX (Invitrogen, NL), vectors containing the trc promoters such as pTrcHisXpressTm (Invitrogen) or pTrc99 (Pharmacia Biotech, SE), or vectors containing the tac promoter such as pKK223-3 (Pharmacia Biotech) or PMAL (new England Biolabs, MA, USA).

Suitable promoting sequences for use with yeast hosts may be regulated or constitutive and are preferably derived from a highly expressed yeast gene, especially a *Saccharomyces cerevisiae* gene. Thus, the promoter of the TRP1 gene, the ADHI or ADHII gene, the acid phosphatase (PH05) gene, a promoter of the yeast mating pheromone genes coding for the a- or α-factor or a promoter derived from a gene encoding a glycolytic enzyme such as the promoter of the enolase, glyceraldehyde-3-phosphate dehydrogenase (GAP), 3-phospho glycerate kinase (PGK), hexokinase, pyruvate decarboxylase, phosphofructokinase, glucose-6-phosphate isomerase, 3-phosphoglycerate mutase, pyruvate kinase, triose phosphate isomerase, phosphoglucose isomerase or glucokinase genes, the *S. cerevisiae* GAL 4 gene, the *S. pombe* nmt 1 gene or a promoter from the TATA binding protein (TBP) gene can be used. Furthermore, it is possible to use hybrid promoters comprising upstream activation sequences (UAS) of one yeast gene and downstream promoter elements including a functional TATA box of another yeast gene, for example a hybrid promoter including the UAS(s) of the yeast PH05 gene and downstream promoter elements including a functional TATA box of the yeast GAP gene (PH05-GAP hybrid promoter). A suitable constitutive PHOS promoter is e.g. a shortened acid phosphatase PH05 promoter devoid of the upstream regulatory elements (UAS) such as the PH05 (-173) promoter element starting at nucleotide -173 and ending at nucleotide -9 of the PH05 gene.

Gene transcription from vectors in mammalian hosts may be controlled by promoters derived from the genomes of viruses such as polyoma virus, adenovirus, fowlpox virus, bovine papilloma virus, avian sarcoma virus, cytomegalovirus (CMV), a retrovirus and Simian Virus 40 (SV40), from heterologous mammalian promoters such as the actin promoter or a very strong promoter, e.g. a ribosomal protein promoter.

Transcription of nucleic acids by higher eukaryotes may be increased by inserting an enhancer sequence into the vector. Enhancers are relatively orientation and position independent. Many enhancer sequences are known from mammalian genes (e.g. elastase and globin). However, typically one will employ an enhancer from a eukaryotic cell virus. Examples include the SV40 enhancer on the late side of the replication origin (bp 100-270) and the CMV early promoter enhancer. The enhancer may be spliced into the vector at a position 5' or 3' to the coding sequence, but is preferably located at a site 5' from the promoter.

Advantageously, a eukaryotic expression vector may comprise a locus control region (LCR). LCRs are capable of directing high-level integration site independent expression of transgenes integrated into host cell chromatin, which is of importance especially where the gene is to be expressed in the context of a permanently-transfected eukaryotic cell line in which chromosomal integration of the vector has occurred, in vectors designed for gene therapy applications or in transgenic animals.

Eukaryotic expression vectors will also contain sequences necessary for the termination of transcription and for stabilising the mRNA. Such sequences are commonly available from the 5' and 3' untranslated regions of eukaryotic or viral DNAs or cDNAs. These regions contain nucleotide segments transcribed as polyadenylated fragments in the untranslated portion of the mRNA .

An expression vector includes any vector capable of expressing nucleic acids that are operatively linked with regulatory sequences, such as promoter regions, that are capable of expression of such DNAs. Thus, an expression vector refers to a recombinant DNA or RNA construct, such as a plasmid, a phage, recombinant virus or other vector, that upon introduction into an appropriate host cell, results in expression of the cloned DNA. Appropriate expression vectors are well known to those with ordinary skill in the art and include those that are replicable in eukaryotic and/or prokaryotic cells and those that remain episomal or those which integrate into the host cell genome.

Construction of vectors according to the invention employs conventional ligation techniques. Isolated plasmids or DNA fragments are cleaved, tailored, and religated in the form desired to generate the plasmids required. If desired, analysis to confirm correct sequences in the constructed plasmids is performed in a known fashion. Suitable methods for constructing expression vectors, preparing in vitro transcripts, introducing DNA into host cells, and performing analyses for assessing expression and function are known to those skilled in the art. Gene presence, amplification and/or expression may be measured in a sample directly, for example, by conventional Southern blotting, Northern blotting to quantitate the transcription of mRNA, dot blotting (DNA or RNA analysis), or in situ hybridisation, using an appropriately labelled probe which may be based on a sequence provided herein. Those skilled in the art will readily envisage how these methods may be modified, if desired.

Vectors as described above may be used in gene therapy techniques and applied to the treatment of diseases. For example, a nucleic acid sequence encoding an antisense molecule according to the present invention may be inserted into a viral or non- viral vector designed for the delivery of nucleic acids to the cells of a patient, either ex-vivo or in vivo.

Examples of viral vectors include adenovirus vectors, adenoassociated virus vectors, retroviral vectors. Examples of non- viral vectors include naked DNA, condensed DNA particles, liposome-type vectors which may include a targeting moiety and, if applicable, escape peptides derived from viruses, and DNA complexed to targeting moieties such as antibodies or cell surface ligands, which are preferably internalised by the target cell.

Agents which are capable of modulating 11β-HSD1 activity are well known in the art. Monder C, White PC. 11 1β-Hydroxysteroid dehydrogenase. Vitamins and Hormones 1993; 47: 187-271, provided an extensive list of such inhibitors in 1993. That list, given as Table IV therein, is incorporated herein by reference. Especially preferred are inhibitors of the reductase activity of 11β-HSD1, which include 11-oxoprogesterone, 3α,17,21-trihydoxy-5β-pregnan-3-one, 21-hydroxy-pregn-4-ene-3,11,20-trione, androst-4-ene-3,11,20-trione and 3β-hydroxyandrost-5-en-17-one.

Further inhibitors, and modes of administration thereof, are known for example from Walker et al., "Carbenoxolone Increases Hepatic Insulin Sensitivity in Man: A Novel Role for 11-oxosteroid Reductase in Enhancing Glucocorticoid Receptor Activation," J. Clin. Endocrinology and Metabolism 80 (11): 3155-59 (1995); Gomez-Sanchez et al., "Central hypertensinogenic effects of glycyrrhizic acid and carbenoxolone," Am J Physiol 263 (6 Pt 1): E1125-E1130 (1992) which showed that liquorice, glycyrrhizic acid, and carbenoxolone were known inhibitors, as well as the infusion of glycyrrhizic acid and carbenoxolone into the lateral ventricle of the brain of the rat at doses less than that which has an effect when infused subcutaneously, produces hypertension, showing that such compounds were administered subcutaneously, orally, and by infusion; see also Whorwood et al., "Liquorice inhibits 11 beta-hydroxysteroid dehydrogenase messenger ribonucleic acid levels and potentiates glucocorticoid hormone action," Endocrinology 132 (6): 2287-92 (1993). Even further still, Homma et al., "A Novel 11β-Hydroxsteroid Dehydrogenase Inhibitor Contained in Saiboku-To, a Herbal Remedy for Steroid-dependent Bronchial Asthma," J. Pharm Pharmacol 46:305-309 (1994), Zhang et al., "Inhibition of 11β-Hydroxysteroid Dehydrogenase Obtained from Guinea Pig Kidney by Furosemide, Naringenin and Some Other Compounds," J Steroid Biochem Molec Biol 49(1):81-85 (1994), and Lee et al., "Grapefruit juice and its flavenoids inhibit 11β-hydroxysteroid dehydrogenase," Clin Pharmacol Ther 59:62-71 (1996), describe even more inhibitors that can be administered in known ways (both in terms of doses and routes of administration), such as flavenoids, which "are sold in tablet form in health food stores and drug stores," and herbs or constituents of herbs. Moreover, Morris et al., "Endogenous 11 beta-hydroxysteroid dehydrogenase inhibitors and their role in glucocorticoid Na+ retention and hypertension," Endocr Res 22(4):793-801 (1996) describe progesterone metabolites as 11β-HSD1 inhibitors, and progesterone is also a substance that can be administered, both in terms of doses and routes of administration, without difficulty by one skilled in the art.

Agents according to the invention may moreover be antibodies. Antibodies, as used herein, refers to complete antibodies or antibody fragments capable of binding to a selected target, and including Fv, ScFv, Fab' and F(ab')₂, monoclonal and polyclonal antibodies, engineered antibodies including chimeric, CDR-grafted and humanised antibodies, and artificially selected antibodies produced using phage display or alternative techniques. Small fragments, such Fv and ScFv, possess advantageous properties for diagnostic and therapeutic applications on account of their small size and consequent superior tissue distribution.

The antibodies according to the invention are especially indicated for diagnostic and therapeutic applications. Accordingly, they may be altered antibodies comprising an effector protein such as a toxin or a label. Especially preferred are labels which allow the imaging of the distribution of the antibody in vivo. Such labels may be radioactive labels or radioopaque labels, such as metal particles, which are readily visualisable within the body of a patient. Moreover, the may be fluorescent labels or other labels which are visualisable on tissue samples removed from patients.

Recombinant DNA technology may be used to improve the antibodies of the invention. Thus, chimeric antibodies may be constructed in order to decrease the immunogenicity thereof in diagnostic or therapeutic applications. Moreover, immunogenicity may be minimised by humanising the antibodies by CDR grafting [see European Patent 0 239 400 (Winter)] and, optionally, framework modification [European Patent 0 239 400; reviewed in international patent application WO 90/07861 (Protein Design Labs)].

Antibodies according to the invention may be obtained from animal serum, or, in the case of monoclonal antibodies or fragments thereof, produced in cell culture. Recombinant DNA technology may be used to produce the antibodies according to established procedure, in bacterial or preferably mammalian cell culture. The selected cell culture system preferably secretes the antibody product.

Therefore, the present invention includes a process for the production of an antibody according to the invention comprising culturing a host, e.g. E. coli or a mammalian cell, which has been transformed with a hybrid vector comprising an expression cassette comprising a promoter operably linked to a first DNA sequence encoding a signal peptide linked in the proper reading frame to a second DNA sequence encoding said protein, and isolating said protein.

Multiplication of hybridoma cells or mammalian host cells in vitro is carried out in suitable culture media, which are the customary standard culture media, for example Dulbecco's Modified Eagle Medium (DMEM) or RPMI 1640 medium, optionally replenished by a mammalian serum, e.g. foetal calf serum, or trace elements and growth sustaining supplements, e.g. feeder cells such as normal mouse peritoneal exudate cells, spleen cells, bone marrow macrophages, 2-aminoethanol, insulin, transferrin, low density lipoprotein, oleic acid, or the like. Multiplication of host cells which are bacterial cells or yeast cells is likewise carried out in suitable culture media known in the art, for example for bacteria in medium LB, NZCYM, NZYM, NZM, Terrific Broth, SOB, SOC, 2 x YT, or M9 Minimal Medium, and for yeast in medium YPD, YEPD, Minimal Medium, or Complete Minimal Dropout Medium.

In vitro production provides relatively pure antibody preparations and allows scale-up to give large amounts of the desired antibodies. Techniques for bacterial cell, yeast or mammalian cell cultivation are known in the art and include homogeneous suspension culture, e.g. in an airlift reactor or in a continuous stirrer reactor, or immobilised or entrapped cell culture, e.g. in hollow fibres, microcapsules, on agarose microbeads or ceramic cartridges.

Large quantities of the desired antibodies can also be obtained by multiplying mammalian cells in vivo. For this purpose, hybridoma cells producing the desired antibodies are injected into histocompatible mammals to cause growth of antibody-producing tumours. Optionally, the animals are primed with a hydrocarbon, especially mineral oils such as pristane (tetramethyl-pentadecane), prior to the injection. After one to three weeks, the antibodies are isolated from the body fluids of those mammals. For example, hybridoma cells obtained by fusion of suitable myeloma cells with antibody-producing spleen cells from Balb/c mice, or transfected cells derived from hybridoma cell line Sp2/0 that produce the desired antibodies are injected intraperitoneally into Balb/c mice optionally pre-treated with pristane, and, after one to two weeks, ascitic fluid is taken from the animals.

The foregoing, and other, techniques are discussed in, for example, Kohler and Milstein, (1975) Nature 256:495-497; US 4,376,110; Harlow and Lane, Antibodies: a Laboratory Manual, (1988) Cold Spring Harbor. Techniques for the preparation of recombinant antibody molecules is described in the above references and also in, for example, EP 0623679; EP 0368684 and EP 0436597.

The cell culture supernatants are screened for the desired antibodies, preferentially by immunofluorescent staining of cells expressing the desired antigen by immunoblotting, by an enzyme immunoassay, e.g. a sandwich assay or a dot-assay, or a radioimmunoassay.

For isolation of the antibodies, the immunoglobulins in the culture supernatants or in the ascitic fluid may be concentrated, e.g. by precipitation with ammonium sulphate, dialysis against hygroscopic material such as polyethylene glycol, filtration through selective membranes, or the like. If necessary and/or desired, the antibodies are purified by the customary chromatography methods, for example gel filtration, ion-exchange chromatography, chromatography over DEAE-cellulose and/or (immuno-)affinity chromatography, e.g. affinity chromatography with an 11β-HSD1 molecule or with Protein-A.

The invention further concerns hybridoma cells secreting the monoclonal antibodies of the invention. The preferred hybridoma cells of the invention are genetically stable, secrete monoclonal antibodies of the invention of the desired specificity and can be activated from deep-frozen cultures by thawing and recloning.

The invention also concerns a process for the preparation of a hybridoma cell line secreting monoclonal antibodies directed to a 11β-HSD1 molecule, characterised in that a suitable mammal, for example a Balb/c mouse, is immunised with a purified 11β-HSD1 molecule, an antigenic carrier containing a purified 11β-HSD1 molecule or with cells bearing 11β-HSD1, antibody-producing cells of the immunised mammal are fused with cells of a suitable myeloma cell line, the hybrid cells obtained in the fusion are cloned, and cell clones secreting the desired antibodies are selected. For example spleen cells of Balb/c mice immunised with cells bearing 11β-HSD1 are fused with cells of the myeloma cell line PAI or the myeloma cell line Sp2/0-Ag14, the obtained hybrid cells are screened for secretion of the desired antibodies, and positive hybridoma cells are cloned.

Preferred is a process for the preparation of a hybridoma cell line, characterised in that Balb/c mice are immunised by injecting subcutaneously and/or intraperitoneally between 10 and 107 and 108 cells of human tumour origin which express 11β-HSD1 containing a suitable adjuvant several times, e.g. four to six times, over several months, e.g. between two and four months, and spleen cells from the immunised mice are taken two to four days after the last injection and fused with cells of the myeloma cell line PAI in the presence of a fusion promoter, preferably polyethylene glycol. Preferably the myeloma cells are fused with a three- to twentyfold excess of spleen cells from the immunised mice in a solution containing about 30 % to about 50 % polyethylene glycol of a molecular weight around 4000. After the fusion the cells are expanded in suitable culture media as described hereinbefore, supplemented with a selection medium, for example HAT medium, at regular intervals.

The invention also provides intracellular antibodies, capable of operating within a cell, for the regulation of 11β-HSD1 levels intracellularly. Intracellular antibodies are advantageously scFv antibodies, expressed intracellularly from expression vectors as is known in the art.

Intracellular antibodies or intrabodies have been demonstrated to function in antigen recognition in the cells of higher organisms (reviewed in Cattaneo, A. & Biocca, S. (1997) Intracellular Antibodies: Development and Applications. Landes and Springer-Verlag). This interaction can influence the function of cellular proteins which have been successfully inhibited in the cytoplasm, the nucleus or in the secretory pathway. This efficacy has been demonstrated for viral resistance in plant biotechnology (Tavladoraki, P., et al. (1993) Nature 366: 469-472) and several applications have been reported of intracellular antibodies binding to HIV viral proteins (Mhashilkar, A.M., et al. (1995) EMBO J 14: 1542-51; Duan, L. & Pomerantz, R.J. (1994) Nucleic Acids Res 22: 5433-8; Maciejewski, J.P., et al. (1995) Nat Med 1: 667-73; Levy-Mintz, P., et al. (1996) J. Virol. 70: 8821-8832) and to oncogene products (Biocca, S., Pierandrei-Amaldi, P. & Cattaneo, A. (1993) Biochem Biophys Res Commun 197: 422-7; Biocca, S., Pierandrei-Amaldi, P., Campioni, N. & Cattaneo, A. (1994) Biotechnology (N Y) 12: 396-9; Cochet, O., et al. (1998) Cancer Res 58: 1170-6).

### Atheroprotective Lipid Profile

Glucocorticoids have been implicated in the development of several metabolic defects found in the Metabolic Syndrome. The importance of pre-receptor metabolism of glucocorticoids is clear for the 11β-HSD2-mineralocorticoid receptor system in the distal nephron (7, 8). Any biological role of 11β-HSD1, which has been proposed to regenerate active corticoids in sites of high expression such as liver, has been obscure. The present invention demonstrates that reduction in 11β-HSD1 levels promotes a 'cardioprotective' plasma lipid and lipoprotein phenotype, at least in part due to changes in expression of key enzymes and transcription factors in the liver.

Distinct phenotypic responses can be defined in the 11 βHSD-1^{-/-} mice, depending on dietary status. *Ad lib* fed 11βHSD-1^{-/-} mice exhibit a 'favourable' lipid profile resulting from an apparent increase in hepatic oxidative drive and reduced levels of several markers associated with increased cardiovascular risk. Fasted 11βHSD-1^{-/-} mice show attenuated glucocorticoid-inducible responses consistent with those observed in their carbohydrate **metabolism (16). Re-feeding after fasting indicates 11βHSD-1^{-/-} mice have increased** hepatic insulin sensitivity. An advantageous metabolic profile is also supported by demonstration of improved glycaemic control in 11βHSD-1^{-/-} mice.

In the *ad lib* fed state, 11βHSD-1^{-/-} mice exhibit several features of a 'cardioprotective' lipid and lipoprotein phenotype. Plasma triglyceride levels are reduced and potentially beneficial HDL cholesterol is elevated. Moreover, 11βHSD-1^{-/-} animals have reduced serum apoCIII. ApoCIII increases plasma triglycerides by inhibiting hepatic lipolysis (38) and interfering with transfer of triglycerides to the liver (34, 39). Reduction of apoCIII would in itself, therefore, contribute to reduced triglycerides. Indeed, apoCIII is positively correlated with cardiovascular disease risk (40). Analogously, null mice show increased ApoAI transcript levels, consistent with raised plasma HDL cholesterol. ApoAI is the main component of HDL and is negatively associated with cardiovascular risk (35).

It is unlikely that decreased synthesis of triglyceride or cholesterol contributes to this phenotype as the expression of key rate-limiting lipogenic and cholesterologenic enzymes was unaffected, consistent with the finding that the lipogenic transcription factor SREBP1c mRNA was also maintained at wild type levels. In contrast, key enzymes of hepatic fatty acid oxidation were elevated in the 11βHSD-1 null mice, compatible with increased hepatic catabolism of triglyceride as a mechanism driving the plasma changes seen.

In contrast, mice which overexpress 11βHSD-1 show the symptoms associated with the metabolic syndrome. Transgenic mice expressing 11βHSD-1 under the control of the adipose-specific adipocyte fatty acid binding protein (aP2) promoter (49) have been created. The transgene-derived transcript was expressed equivalently in adipose tissue from subcutaneous abdominal, epididymal, mesenteric, and interscapular brown adipose tissue (BAT) depots but was absent in brain, liver, skeletal muscle, and kidney of transgenic (Tg) mice. The levels of overexpression of 11βHSD-1 were similar to those observed in human patients suffering from metabolic syndrome. These mice had increased adipose levels of corticosterone and developed visceral obesity that was exaggerated by a high-fat diet. The mice also exhibited pronounced insulin-resistant diabetes, hyperlipidemia, and, surprisingly, hyperphagia despite hyperleptinemia.

Similarly, mice overexpressing 11βHSD-1 under the control of the liver-specific Apo-E promoter have been created to study overexpression of 11βHSD-1 in the liver. These mice exhibit at least 5-fold more 11β-HSD1 activity in the liver than wild type mice. Analyses show male mice have normal glucose tolerance, suggesting that 11β-HSD1 is not limiting for glucose homeostasis under basal conditions. However, transgenic animals show increased plasma insulin levels (~50%), elevated plasma triglycerides (~2 fold) and increased plasma total cholesterol (20%) largely attributable to the non-HDL fraction (HDL-cholesterol/total = 0.62 transgenic vs 0.83 wild type). Histological analyses reveal accumulation of lipid in hepatocytes of transgenic mice, suggesting lipid metabolism may be differentially sensitive to increased GC regeneration in the liver. In addition, male transgenic mouse body weight is modestly elevated compared to wild type littermates (6-8%), evident from ~10 weeks of age. The data indicate that selective increases in liver 11β-HSD1 modestly reduce insulin sensitivity, disadvantageously elevate plasma lipids and produce hepatic lipid accumulation, thus manifesting some, but not all aspects of the metabolic syndrome. The interplay between liver and adipose GC levels, determined by local 11β-HSD1, appear crucial in determining the manifestations of the Metabolic Syndrome.

### PPARα

The increased triglyceride catabolism observed in 11βHSD-1^{-/-} mice may stem from elevated PPARα levels; this is consistent with reports that the genes of fatty acid oxidation CPT-I (31), ACO (32), as well as UCP-2 (30, 33) are targets for PPARα in liver.

Indeed, a number of changes observed in the 11βHSD-1^{-/-} mice suggest elevated PPARα levels may play a functional role in the atheroprotective phenotype. Thus, PPARα activation by fibrate ligands lowers plasma triglyceride and represses apoCIII (23) and Aα-fibrinogen expression (41). The 25% reduction in Aα-fibrinogen transcript levels observed in the 11βHSD-1^{-/-} mice is similar to the effect of fibrate administration and is consistent with this transcript being PPARα repressible (41). Since changes in Aα-transcript levels closely follow changes in plasma levels (41) we infer that the reduced transcript levels observed here would be likely to contribute to the overall atheroprotective profile of the 11βHSD-1^{-/-} mouse. High fibrinogen levels are independently correlated with increased cardiovascular risk (37). It could be said, therefore, that the fed 11βHSD-1^{-/-} animals mimic in part the phenotype of a fibrate treated animal.

11βHSD-1 null mice show apparently lower intracellular glucocorticoid levels and action in the face of elevated basal and post-stress (e.g. fasting) plasma corticosterone levels (16). This underlines the importance of regeneration of corticosterone from 11-dehydrocorticosterone in determining intracellular glucocorticoid effects. The lack of induction of PPARα with fasting is compatible with this notion, but it cannot explain the elevated fed PPARα levels. PPARα is induced by glucocorticoids (18) and follows a diurnal cycle that parallels the corticosterone rhythm (19). This implies that control of PPARα expression by glucocorticoid occurs not only in extreme conditions such as the stress-response to fasting but also during the normal diurnal cycle where glucocorticoid and insulin levels show more modest changes. One potential explanation for elevated PPARα expression at the diurnal nadir (8am) in 11βHSD-1^{-/-} mice is that they have subtly elevated plasma corticosterone levels at this time (this study: wild type 25.2±7.2nmol/l versus 11βHSD-1^{-/-} 47.5±7.8 nmol/L, p<0.05, in good agreement with our previous reports (16, 24). This results from somewhat impaired negative feedback upon the HPA axis normally amplified by 11βHSD-1 (16, 24). Interestingly, 11β-HSD-1^{-/-} mice show a reduced intracellular glucocorticoid response in brain in the face of an exaggerated stress-mediated increase in plasma corticosterone (42). This would imply that liver gene expression is perhaps less sensitive to the exquisite regulation of gene expression mediated by 11βHSD-1 in the brain and is more sensitive to the prevailing plasma corticosterone levels. However, levels of the glucocorticoid-sensitive hepatic binding protein CBG and liver GR binding are similar (24) in *ad lib* fed 11βHSD-1^{-/-} mice and wild type mice in the morning. The lack of down-regulation of GR (43) and CBG (44) in 11βHSD-1^{-/-} liver, in the face of elevated plasma corticosterone levels indicate that effective glucocorticoid action within the liver is indeed attenuated, suggesting that factors other than merely plasma corticosterone concentrations are responsible for the increased hepatic PPARα expression. PPARα is regulated by myriad factors including other steroids (45), lipids (46), retinoids (47) and hormones (48), including insulin as shown in the present study. The precise determinants of elevated basal PPARα in this model of chronic subtle glucocorticoid depletion in the liver remain to be determined.

It is also clear that PPARα and GR have overlapping and sometimes opposing actions on target promoters. For example, fibrinogen levels are positively regulated by glucocorticoids (36, 49) and negatively regulated by PPARα (41). Similarly, apolipoprotein AI is induced by glucocorticoids (50) whereas in mice apoAI (23), and apoAII levels are repressed by PPARα. Our observation of elevated ApoAI transcript levels in fed 11βHSD-1^{-/-} mice could imply that the apoAI promoter is more sensitive to glucocorticoid-mediated induction than to PPARα-mediated repression. For some promoters the GR effect seems to predominate, for others PPARα. Alternatively, since insulin is known to positively regulate the apoAI promoter (85), increased insulin sensitivity in 11βHSD-1^{-/-} liver mice may also explain the discrepancy in gene expression observed. Further work will be necessary to determine the underlying mechanism for the apoAI expression pattern. However we would expect that since this component of the HDL reverse cholesterol transport system is negatively correlated with cardiovascular risk that elevated levels could contribute to the overall atheroprotective profile of 11βHSD-1^{-/-} mice.

### PPARγ

PPARγ is important in mediating macrophage uptake and export of oxidised or acetylated LDL-cholesterol, notably in the atheromatous plaque in blood vessel walls. The dominant effect appears to be on cholesterol export via ABCA1 and related transporters (75, 76). 11β-HSD1 inhibition therefore facilitates cholesterol export from macrophages in the plaque by increasing expression of PPARγ, reducing foam cell formation and their contribution to atherogenesis.

11β-HSD1 knockout mice show increased levels of macrophage PPARγ expression, showing that the use of PPARγ agonists such as thiazolidinediones and N-(2-benzoylphenyl) tyrosine analogues is effective in reducing cholesterol storage in macrophages and the formation of foam cells.

Available PPAR agonist drugs, such as rosiglitazone and pioglitazone, are indicated for treating insulin resistance in type 2 diabetes. Increased levels of PPARγ expression are also seen in adipose tissue of 11β-HSD1 knockout mice, indicating that the combination of 11β-HSD1 inhibitors and PPARγ agonists is effective in treating insulin sensitivity and other effects of the metabolic profile, such as plasma lipid profile, glucose tolerance and cardiovascular risk. Moreover, since the side effects of treatment with PPARγ agonists such as thiazolinediones include weight gain, combination therapy with an 11β-HSD1 inhibitor is indicated in order to control the increase in adipose tissue associated with PPARγ agonist therapy.

### Glucose Metabolism

Among the physiological roles of glucocorticoids is the adaptation of animals to prolonged nutrient deprivation. During this response, elevated glucocorticoid levels drive increased hepatic glucose production and fatty acid oxidation whilst concomitantly facilitating adipose tissue lipolysis to provide the fatty acids and glycerol required by the liver. In the fasted state, 11β-HSD-1^{-/-} mice show attenuation of glucocorticoid-sensitive gene expression. PPARα and apoAI show attenuated induction whereas GPAT exhibits an attenuated fasting repression. These results are in agreement with previous findings on attenuation of glucocorticoid-inducible glucose metabolism in 11β-HSD-1^{-/-} mice (16). This implies that the null mice have a relative lack of intracellular glucocorticoid during fasting or stress. Despite this attenuated induction, the mice appear to be capable of maintaining their hepatic fatty acid oxidation system over a 24 hour fast. Thus, despite an abolished fasting induction of PPARα in11β-HSD-1^{-/-} mice, a major rate limiting enzyme in mitochondrial oxidation (CPT-1) appears to be normally induced, and fasting plasma glucose levels are not significantly lower than wild type animals. This is in contrast to findings in fasted PPARα null mice which exhibit profound hypoglycaemia upon prolonged fasting (20, 21). There is relatively pronounced lipid accumulation in 11βHSD-1^{-/-} liver on fasting, reminiscent of the fatty liver observed in fasted PPARα null mice (20, 21). However, lipid accumulation seems to resolve in 11β-HSD-1^{-/-} mice upon re-feeding. Whether lipid accumulation is due to blunted PPARα-driven increases in fatty acid oxidation, as in fasted PPARα knockout mice, remains to be determined. Indeed, whilst PPARα may regulate CPT-I levels in the *ad lib* state (31), fast-mediated induction of CPT-I is unaffected in PPARα knockout mice (83) implying that this process is independent of the transcription factor. An alternative explanation could come from our observation of attenuated glucocorticoid-mediated fasting repression of the lipid esterification enzyme GPAT. Elevated levels of such a rate limiting enzyme in the lipid synthesis pathway could contribute to the lipid accumulation observed. Indeed, raised GPAT levels may also partly account for the lower fold reduction in plasma triglyceride on fasting in null mice compared to wild type. Since GPAT is insulin-inducible, this finding is also consistent with the growing evidence that 11βHSD-1^{-/-} liver is more sensitive than wild type to even the extremely low insulin levels found during fasting. The PPARα-sensitive ACO and UCP-2 transcripts show attenuated induction with fasting and may reflect the relatively greater sensitivity of these promoters, compared to that of CPT-I, to PPARα regulation on fasting. Partial induction of downstream target genes by PPARα in the face of a blunt fasting increase in PPARα levels could mean that activation of the already elevated 11 βHSD-1^{-/-} levels of PPARα within a 24-hour fasting period is sufficient to promote an adaptive metabolic response in 11βHSD-1^{-/-} mice. This is a possibility since endogenous fatty acids activate PPARα (84) and there is an increased provision of fatty acid to the liver during a fast. Alternatively, other processes may elevate expression of the oxidative enzymes during fasting (83).

Re-feeding after a fast is characterised by a pronounced insulin-mediated overshoot in liver gene expression of enzymes in the lipogenic pathways and repression of oxidative processes. We have used this as a measure of hepatic insulin sensitivity. 11βHSD-1^{-/-} mice clearly have increased hepatic insulin sensitivity since on refeeding there is an exaggerated suppression (CPT-I, UCP-2) or induction (SREBP-1c, FAS, GPAT, HMGCoA-R) of transcript levels for oxidative and lipogenic enzymes, respectively. Pronounced induction of the lipogenic pathway (SREBP-1c, FAS and GPAT) combined with an exaggerated repression of oxidative lipid metabolism (CPT-I, UCP-2) upon refeeding after fast may also account for the rapid return of triglycerides to *ad lib* fed values by 4h and the overshoot of plasma triglycerides seen at the 24 hour re-feeding period in 11βHSD-1^{-/-} mice. The contention of increased insulin sensitivity is supported by intraperitoneal glucose tolerance tests that show 11βHSD-1^{-/-} mice have improved glycaemic control. However, muscle is the major post-prandial site of glucose disposal, and it is unclear whether improved insulin sensitivity in the liver of the 11βHSD-1^{-/-} mice can account entirely for the improved glucose tolerance. Direct studies on 11βHSD-1^{-/-} mouse muscle are required to address this issue. Clearly, since insulin resistance is one of the major underlying mechanisms ascribed to the pathogenic development of the metabolic syndrome, demonstration of increased hepatic insulin sensitivity and improved glucose tolerance can be interpreted as beneficial.

Mice with a targeted disruption in the gene encoding the 11βHSD-1 enzyme represent a model animal that lacks a crucial intracellular glucocorticoid re-amplifying mechanism. 11βHSD-1^{-/-} mice resist hyperglycaemia upon stress and obesity (16) and have a favourable metabolic and lipidaemic profile due to altered expression and activity of liver proteins. However, 11βHSD-1 is also expressed in other tissues such as fat and brain, important sites regulating lipid and nutrient homeostasis. 11βHSD-1 may also, therefore, modulate glucocorticoid action on central energy balance as well as peripheral fat storage, insulin action and glucose tolerance. These effects cannot be ruled out as having a bearing on the lipid profile, in combination with the hepatic effects of 11βHSD-1 knockout on lipid metabolism described here. Nevertheless, the improved fed and re-fed metabolic profiles in the 11βHSD-1 null mice suggest inhibitors of this enzyme may have favourable effects on several cardiovascular risk factors. This is particularly pertinent as the expression of the enzyme in liver was unaffected by the dietary manipulations *in vivo,* suggesting that the putative drug target is maintained. Further, a combination of an 11βHSD-1 inhibitor and a PPARα agonist represents an extremely powerful therapeutic strategy for treating dyslipidaemias, glucose intolerance and hyperfibrinogenaemia.

### Carbenoxolone improves lipid profile in obese rats

The effects of inhibition of 11β-HSDs with carbenoxolone has been examined in obese Zucker rats, a strain in which tissue-specific dysregulation of 11β-HSD1 (increased in adipose, decreased in liver) mirrors changes in human obesity (4; 3; 54). Obesity in these animals is associated with increased 11 β-HSD1 in adipose tissue and down-regulation in liver. As in lean rats, carbenoxolone was effective in inhibiting 11β-HSD1 activity in liver and 11 β-HSD2 activity in kidney. This illustrates that further reduction in hepatic 11β-HSD1 activity can be achieved pharmacologically in obese animals, beyond their basal down-regulation of enzyme expression and activity. Carbenoxolone had no effect on fasting plasma glucose or glucose tolerance, as in lean rats. However, in the obese rats carbenoxolone did induce the same pattern of altered lipid profile (with decreased triglycerides and increased HDL cholesterol) which has been observed in the 11β-HSD1 knockout mouse (55). In the mouse model, this has been attributed to enhanced hepatic lipid oxidation, and probably results from up-regulation of PPARα in liver (55).

Inhibition of 11β-HSD1 with carbenoxolone in liver thus has beneficial effects on lipid metabolism in Zucker obese rats, despite lower basal 11β-HSD1 'target' activity.

### Reducing intrahepatic fat content

Studies in ZIP1-fatless mice have demonstrated that ectopic fat accumulation in the liver and in skeletal muscle is associated with severe insulin resistance and signalling defects such as defects in insulin-stimulated IRS-1 and IRS-2-associated PI 3-kinase activity(50). Treatment of lipodystrophy in these mice with fat transplantation completely reverses insulin resistance (51). In humans, ectopic fat accumulation in the liver is also associated with hepatic insulin resistance independent of body weight and alcohol consumption (52; 53).

11βHSD-1 amplifies local glucocorticoid action by catalysing the intracellular conversion of inactive cortisone to active cortisol particularly in the liver. 11β-HSD1 deficient mice have markedly low serum triglyceride concentrations, reduced gluconeogenic responses to stress or fat feeding, and enhanced hepatic insulin sensitivity. These data demonstrate that local glucocorticoid production modulates insulin action in the rodent liver. In humans, inhibition of 11β-HSD1 by carbenoxolone increases the amount of glucose required to maintain normoglycemia without altering forearm glucose uptake, suggesting enhanced hepatic insulin sensitivity.

Men with a relatively normal body weight were studied to determine whether liver fat content, as measured using proton spectroscopy is associated with features of insulin resistance independent of body weight. It has been found that liver fat content is an independent determinant of the sensitivity of endogenous glucose production to insulin. A high liver fat content was also associated, independent of body weight and visceral adipose tissue, with several facets of insulin resistance including hyperinsulinemia, hypertriglyceridemia and a slightly increased ambulatory systolic blood pressure.

Impaired suppression of serum FFA was observed in men with high vs. low liver fat, again independent of all measures of overall adiposity. None of the characteristic changes of the HPA axis which occur with obesity were observed in men with high liver fat. However, these men converted more oral cortisone acetate to serum cortisol during suppression of endogenous cortisol secretion, suggesting increased liver 11β-HSD-1 activity.

Fatty liver, together with adverse lipid profiles and insulin resistance, are also observed in ApoE - 11β-HSD1 liver-specific knock-in mice, which express elevated levels of 11β-HSD1 in the liver. Thus, inhibition of 11β-HSD1 in the liver reduce intrahepatic fat content and thereby improve lipid profile, lowering triglycerides and elevating HDL cholesterol levels, enhance insulin sensitivity, and reduce elevated transaminases and reduce the chance of progression to non-alcoholic steatohepatitis or cirrhosis. The antidiabetic drug metformin reduces glucocorticoid receptor levels in the liver, indicating that coadministration of metformin and an 11β-HSD1 inhibitor is synergistic.

### Administration

Agents according to the invention may be delivered by conventional medicinal approaches, in the form of a pharmaceutical composition. A pharmaceutical composition according to the invention is a composition of matter comprising a combination of a PPARα agonist and an 11βHSD-1 inhibitor. The active ingredient(s) of a pharmaceutical composition according to the invention is contemplated to exhibit excellent therapeutic activity, for example, in the alleviation of cardiovascular diseases. Dosage regima may be adjusted to provide the optimum therapeutic response. For example, several divided doses may be administered daily or the dose may be proportionally reduced as indicated by the exigencies of the therapeutic situation.

The active compound may be administered in a convenient manner such as by the oral, intravenous (where water soluble), intramuscular, subcutaneous, intranasal, intradermal or suppository routes or implanting (e.g. using slow release molecules). Depending on the route of administration, the active ingredient may be required to be coated in a material to protect said ingredients from the action of enzymes, acids and other natural conditions which may inactivate said ingredient.

In order to administer the combination by other than parenteral administration, it will be coated by, or administered with, a material to prevent its inactivation. For example, the combination may be administered in an adjuvant, co-administered with enzyme inhibitors or in liposomes. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether. Enzyme inhibitors include pancreatic trypsin.

Liposomes include water-in-oil-in-water CGF emulsions as well as conventional liposomes.

The active compound may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene gloycol, and the like), suitable mixtures thereof, and vegetable oils. The proper fluidity can be maintained, for example, by the use of a coating such as lecithin, by the maintenance of the required particle size in the case of dispersion and by the use of superfactants.

The prevention of the action of microorganisms can be brought about by various antibacterial and antifungal agents, for example, parabens, chlorobutanol, phenol, sorbic acid, thirmerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilisation. Generally, dispersions are prepared by incorporating the sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the combination of polypeptides is suitably protected as described above, it may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. The amount of active compound in such therapeutically useful compositions in such that a suitable dosage will be obtained.

The tablets, troches, pills, capsules and the like may also contain the following: a binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier.

Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier and/or diluent" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such as active material for the treatment of disease in living subjects having a diseased condition in which bodily health is impaired.

The principal active ingredients are compounded for convenient and effective administration in effective amounts with a suitable pharmaceutically acceptable carrier in dosage unit form. In the case of compositions containing supplementary active ingredients, the dosages are determined by reference to the usual dose and manner of administration of the said ingredients.

The invention is further described below, for the purpose of illustration, in the following examples.

### Examples

### Experimental Procedures - 11βHSD-1 knockout mice

*Animals*- Male wild type MF1 11βHSD-1^{-/-} mice and their controls, bred as previously described (16), are housed in standard conditions on a 12h light: 12h dark cycle (lights on 7am). For experiments, animals are housed singly and allowed to acclimatise for at least two days. Animals are allocated at random (n=6 per group) to receive either ad lib access to chow, a 24 h fast, a 24 h fast with a 4 h re-feed or a 24 h fast with a 24 h re-feed. All fasting commenced at 8am. Animals are killed by decapitation in a separate room from their housing within 1 min of their cage being disturbed.

Groups of eight 5-week-old male obese and lean Zucker rats (Harlan Orlac, Bicester, UK) were characterised by phenotype; maintained under controlled conditions of light (on 0800 h - 2000 h) and temperature (21°C) and allowed free access to standard rat chow (Special Diet Services, Witham, UK) and drinking water.

*Plasma Parameters* -Trunk blood is collected rapidly, plasma separated and samples kept on ice until measurement of triglyceride, free fatty acids, total cholesterol and HDL cholesterol. Triglyceride is measured using a lipase based colourometric TG kit (Roche, Mannheim, GmbH). Total and HDL cholesterol are measured using the CHOL and HDL C-Plus kits (Roche). Glucose is measured with a Glucose HK kit (Sigma, Poole, UK). Plasma insulin is measured using an ELISA performed according to manufacturers instructions (Crystalchem, Chicago, USA). Corticosterone levels are determined by radioimmunoassay, as described (24) Serum is also obtained and analysed for true triglyceride (glycerol-free peak by FPLC separation) and cholesterol profiles by FPLC followed by enzymatic methods as previously described (23). ApoAI, apoAII, apoCII, apoB and apoE are measured by nephelometry using specific antibodies on representative samples.

*RNA Extraction and Analysis* - Tissues are snap-frozen in liquid nitrogen and homogenised in Trizol (Gibco BRL, Paisley, UK). Total RNA is purified by adding a binding matrix (Rnaid Plus kit, BIO 101, Anachem, UK) and eluted from the matrix in diethylpyrocarbonate (Sigma) pre-treated water containing 400units per ml RNAsin (Promega, Southampton, UK) and 10mM DTT. RNA (5-20µg) is resolved on a 1% MOPS formaldehyde gel and blotted according to standard northern blot procedure in 20x SSC onto Hybond N⁺ membranes (Amersham, Little Chalfont, UK). Probes are labelled with ^{32P}d-CTP using a random primed labelling kit (Roche), purified through Nick Columns (Pharmacia-Amersham, Little Chalfont, UK) and hybridised overnight in high SDS (6%) phosphate buffer (0.2M NaH₂PO₄, 0.6M Na₂HPO₄, 5mM EDTA) containing 0.5mg/ml denatured salmon testes DNA (Sigma) at 65°C. Blots are washed at 65°C to a maximum stringency of 0.5xSSC, 0.1%SDS, exposed to phosphor imager film (FLA2000, Fujifilm, London, UK) and analysed by quantitative phosphor imager software (Aida, Raytek Scientific, Sheffield, UK). Blots are also exposed to film (biomax-MR, Kodak, UK). The probes used for this study are detailed in ref. 55 and are generated from primers designed to sequences in Genbank. All probe identities are confirmed by sequencing using the Thermosequenase kit (USB, Cleveland, USA) on standard 8% acrylamide sequencing gels.

*Intraperitoneal Glucose Tolerance Test*- In a separate experiment, transgenic and wild type mice are fasted overnight and then injected intraperitoneally with 2mg/g D-glucose (25% stock solution in phosphate buffered saline). Blood samples are taken by tail venesection into EDTA-micro tubes (Sarstedt, Leicester, UK) at zero (before injection and within 1 minute of disturbing the cage) and at 5, 15 30 60 and 120 minute intervals after the glucose load.

*11β-HSD-1 Enzyme Activity -* Liver samples are homogenised as described (12). The reaction included 0.1 mg/ml protein, 25 nM tritiated corticosterone and an excess (2µM) of the 11βHSD-1 specific co-factor NADP (under *in vitro* conditions in homogenised tissues, 11β-HSD1 is bi-directional, with assay of dehydrogenation more stable). The assay is in the linear range of protein concentration and product formation. After a 10 min incubation, steroids are extracted with ethylacetate. Steroids are separated by thin layer chromatography (TLC), identified by migration in comparison to unlabelled corticosterone and 11-dehydrocorticosterone standards and quantified with a phosphorimager tritium screen (Fujifilm). TLC results are validated by HPLC analysis of a representative group of samples from each experimental group.

### Experimental techniques - Zucker rats

*Drug treatment -* Drug treatment was commenced when the animals were 6 weeks of age. Carbenoxolone (50mg/kg body weight) or a matched volume of vehicle (water; 1ml/kg/day) was administered by gavage daily at 0900h. Animals were weighed regularly to allow accurate dosing with drugs, and to follow the progress of weight gain. Food intake for each cage of 4 animals was measured daily. After two weeks of treatment animals underwent an oral glucose tolerance test, which consisted of an overnight fast, followed by an oral glucose load of 2g/kg body weight at 0900h. Blood samples were taken by tail-nick at 0, 30 and 120 min after the glucose bolus. At nine weeks of age, after three weeks of carbenoxolone or vehicle treatment, animals were decapitated at 0900-1100h, trunk blood collected and tissues dissected and either snap-frozen on dry ice or mechanically homogenised in Krebs bicarbonate Ringer buffer (118mM NaCl, 3.8mM KCl, 1.19mM KH₂PO₄, 2.54mMCaCl₂, 1.19mM MgSO₄, 25mM NaHCO₃, pH=7.4).

*Plasma assays* - Corticosterone levels were measured in plasma prepared from terminal blood samples collected at 0900 - 1100h using an in-house radioimmunoassay (74). The inter- and intra-assay coefficients of variation were <10%.

Glucose concentrations were determined using a hexokinase glucose assay kit (Sigma, Poole, UK), for which the inter- and intra-assay coefficients of variation were both <2%. Insulin was measured using a rat [¹²⁵I]-insulin radioimmunoassay kit (Amersham, Buckinghamshire, UK), for which the inter- and intra-assay coefficients, of variation were <15 and <10% respectively.

Lipid levels were measured in plasma prepared from terminal blood samples collected at 0900 - 1100h. Triglycerides, total cholesterol and HDL cholesterol were measured using ELISA kits (TG, CHOL and HDL C-plus respectively) from Roche Diagnostics, Mannheim, Germany. Non-esterified fatty acids (NEFAs) were measured using the Wako NEFA C enzymatic assay (Alpha Laboratories Ltd, Hampshire, UK).

*Measurement of enzyme activities* in vitro - 11β-HSD1 activity was measured in homogenates of tissues by incubating in duplicate at 37°C, in Krebs Ringer buffer containing glucose (0.2%), NADP (2 mM) and 1,2,6,7-[³H]₄-corticosterone (100 nM). Conditions were optimised for each tissue to ensure first order kinetics, by adjusting protein concentrations as follows: 10 µg/ml for liver; 1.5 mg/ml for quadriceps skeletal muscle; 0.5 mg/ml for subcutaneous lumbar fat and 1 mg/ml for omental fat. After 60 min incubation, steroids were extracted with ethyl acetate, the organic phase evaporated under nitrogen and extracts re-suspended in mobile phase (20% methanol, 30% acetonitrile and 50% water). Steroids were separated by HPLC using a reverse phase µ-Bondapak C18 column at 20°C and quantified by on-line liquid scintillation counting. No peaks other than ³H-corticosterone and ³H-11-dehydrocorticosterone were detected under these conditions.

11β-HSD2 activity in the kidney was determined in a similar way, with homogenates (protein concentration SOµg/ml) incubated with NAD (2 mM) as cofactor and 10nM [³H]-corticosterone. Steroids were extracted with ethyl acetate and separated by HPLC as above.

5β-Reductase activity in the liver was assessed by the conversion of [³H]-corticosterone to [³H]-5β-tetrahydrocorticosterone in liver cytosol preparations. The sub-cellular localisation and cofactor preference of 11β-HSD1 and 5β-reductase differ such that the enzyme activities can be measured independently of one another. Liver cytosol was prepared by repeated centrifugation according to the method of (Fleischer & Kervina 1974). Enzyme activity was measured by incubating cytosol (100µg protein/ml) in duplicate at 37°C, in phosphate buffer (40mM Na₂PO₄, 320mM sucrose, 1 mM dithiothreitol, pH 7.5) containing NADPH (1 mM) and [³H]-corticosterone (150 nM). Incubations were carried out for 60min, following which steroids were extracted with ethyl acetate, the organic phase evaporated under nitrogen and extracts re-suspended in mobile phase (25% methanol, 10% acetonitrile and 65% water). Steroids were separated by HPLC using a reverse phase C8 column at 10°C, and quantified by on-line liquid scintillation counting. Under these conditions production of ³H-11-dehydrocorticosterone was below the limit of detection (i.e. <2%).

Radiolabelled-steroids were from Amersham (Bucks, UK). Solvents were HPLC glass-distilled grade from Rathburn Chemicals (Walkerburn, UK). Other chemicals were from Sigma (Poole, UK).

*Statistics -* All data are expressed as mean ± standard error. Data were analysed by Analysis of Variance followed by post hoc least squares difference tests. N=8 for all groups.

### Experimental techniques-Hepatic fat accumulation

*Subjects and study design -* Healthy men were recruited from occupational health services in Helsinki. The subjects were healthy as judged by history and physical examination, and did not use any drugs. The subjects did not have serological evidence of hepatitis A, B or C, or of autoimmune hepatitis, nor did they show clinical signs or symptoms of inborn errors of metabolism or a history of use of toxins or drugs associated with steatosis ¹⁵. The subjects were divided into 'high LFAT' and 'low LFAT' groups based on their median LFAT (5 %). As detailed below, the subjects underwent measurements of i) conversion of cortisone to cortisol *in vivo* ii) *in vivo* insulin sensitivity of glucose Rₐ and R_{d} using the euglycemic insulin clamp technique combined with infusion of [3-³H]glucose, iii) liver fat content by proton spectroscopy, iv) s.c., visceral and total fat volumes by MRI, v) VO₂max, and vi) 24-hour ambulatory blood pressure.

The purpose, nature, and potential risks of the studies were explained to the subjects before their written informed consent was obtained. The experimental protocol was approved by the ethical committee of the Helsinki University Hospital.

*Cortisol secretion and metabolism -* Conversion of cortisone to cortisol by 11β-HSD-1 on first pass through the liver was measured after the subjects took 1 mg oral dexamethasone at 11 p.m. to suppress endogenous cortisol production and fasted overnight (3; 54; 56) . The following morning, a catheter was inserted into an antecubital vein for blood sampling and the subjects ingested 25 mg cortisone acetate. Serum cortisol was then measured every 15 min for 90 min.

*In vivo insulin sensitivity of glucose production and utilisation -* At 8 a.m. after an overnight fast, two indwelling catheters were placed, one in an antecubital vein and one retrogradely in a heated hand vein, for infusion of glucose, insulin and [3-³H]glucose and for sampling of arterialized venous blood. To determine rates of glucose production (Rₐ) and utilization (R_{d}) under basal and hyperinsulinemic conditions, [3-³H]glucose was infused in a primed (20 µCi) continuous (0.2 µCi/min) fashion for a total of 300 min (52; 58). Baseline blood samples were taken for measurement of fasting serum insulin and glucose concentrations and for the biochemical measurements listed in Figure 14. After 120 minutes, insulin was infused in a primed-continuous (0.3 mU/kg·min) fashion as previously described (52). Plasma glucose was maintained at 5 mmol/l (90 mg/dl) until 300 min using a variable rate infusion of 20 % glucose (58). Blood samples for measurement of glucose specific activity were taken at 90, 100, 110 and 120 min and at 15-30 min intervals between 120 and 300 min. Serum free insulin concentrations were measured every 60 min intervals during the insulin infusion.

[3-³H]glucose specific activity was determined as previously described (59). Glucose Rₐ and R_{d} were calculated using the Steele equation, assuming a pool fraction of 0.65 for glucose and distribution volume of 200 ml/kg for glucose (60). Endogenous glucose Rₐ was calculated by subtracting the exogenous glucose infusion rate required to maintain euglycemia during hyperinsulinemia from the rate of total glucose Rₐ. The % suppression of basal endogenous glucose Rₐ during the last hour (240-300 min) by insulin was used as an index of the sensitivity of endogenous glucose production to insulin (% suppression).

*Liver fat content (proton spectroscopy) -* Single voxel (2 x 2 x 2 cm³) proton spectra from the liver were acquired using 32 excitations, a loop surface coil and a 1.5 T magnetic resonance device (Magnetom Vision, Siemens, Erlangen, Germany). Spatial location was achieved by using a stimulated echo acquisition mode applied with a repetition time of 3000 ms and with an echo time of 20 ms. A long repetition time and short echo time were chosen to minimise effects of T1 and T2 relaxation, respectively, on signal intensities. Chemical shifts were measured relative to water signal intensity at 4.8 ppm (S_{water}). Methylene signal intensity, which represents intracellular triglycerides in the liver (52), was measured at 1.4 ppm (S_{fat}). Signal intensities were obtained by a time domain fitting routine VAPRO-MRUI (www.mrui.uab.es/mruiHomePage.html). This measurement of % hepatic fat by proton spectroscopy has been validated against the lipid content of liver biopsies in humans (61). It has also been validated against liver density measurements performed by computed tomography. The latter validation has also been performed by us previously (52). Hepatic fat % was calculated by dividing 100 times S_{fat} by the sum of S_{fat} and S_{water}.

*Intra-abdominal fat (magnetic resonance imaging) -* A series of T1-weighted trans-axial scans for the determination of visceral and subcutaneous fat were acquired from a region extending from 4 cm above to 4 cm below the 4^{th} and 5^{th} lumbar interspace (16 slices, field of view 375 x 500 mm², slice thickness 10 mm, breath-hold repetition time divided by the echo time 138.9 ms/4.1 ms). Visceral and subcutaneous fat areas were measured using an image analysis program (www.perceptive.com/ALICE.HTM). A histogram of pixel intensity in the intra-abdominal region was displayed and the intensity corresponding to the nadir between the lean and fat peaks was used as a cutpoint. Visceral adipose tissue was defined as the area of pixels in the intra-abdominal region above this cutpoint. For calculation of subcutaneous adipose tissue area, a region of interest was first manually drawn at the demarcation of subcutaneous adipose tissue and visceral tissue as previously described (52).

*Maximal aerobic power (VO₂max)* - Maximal aerobic power was measured directly using an incremental work-conducted upright exercise test with an electrically braked cycle ergometer (Ergometer Ergoline 900ERG, Germany) combined with continuous analysis of expiratory gases and minute ventilation (Vmax229 series, SensorMedics). Exercise was started at a work load of 50 watts. The work load was then increased by 50 watts every 3 min until perceived exhaustion or a respiratory quotient of 1.10 was reached. Maximal aerobic power was defined as the VO₂max of the last 30 s of exercise.

*24-h ambulatory blood pressure -* Noninvasive ambulatory blood pressure monitoring was performed on a normal weekday with an automatic ambulatory blood pressure monitoring device (Diasys Integra, Novacor, France). The device was set to record blood pressure and heart rate every 15 minutes during daytime and every 30 minutes during night-time. Day and night were defined from the waking and sleeping periods in the subject's diary.

*Other measurements -* Plasma glucose concentrations were measured in duplicate with the glucose oxidase method using Beckman Glucose Analyzer II (Beckman Instruments, Fullerton, CA) (63). HbA_{1c} was measured by high pressure liquid chromatography (62) using the fully automated Glycosylated Hemoglobin Analyzer System (BioRad, Richmond, CA). Total cholesterol, HDL cholesterol, and triglycerides were measured as previously described (64). Serum free insulin was measured using radioimmunoassay (Pharmacia Insulin RIA kit, Pharmacia, Uppsala, Sweden) after precipitation with polyethylene glycol (65). Serum FFA were measured using a fluorometric method (66). The % body fat was measured by bioimpedance plethysmography (Bio-Electrical Impedance Analysis System, Model #BIA-101A, RJL Systems,MI) (67).

*Statistical analyses* - The unpaired t-test was used to compare mean values between low and high LFAT groups. The factors explaining variation in serum cortisol concentrations after oral cortisol was analysed using ANOVA for repeated measures and multiple linear regression analysis. The calculations were made using the Systat statistical package, version 10.0 (Systat, Evanston, IL) and GraphPad Prism version 2.01 (GraphPad Inc, San Diego, CA). All data are shown as mean±standard error of mean. A p-value less than 0.05 was considered statistically significant.

### Example 1

### 11βHSD-1^{-/}⁻ mice have lower plasma triglyceride and higher HDL cholesterol

Plasma triglycerides are lower in ad lib fed 11βHSD-1 null mice (Fig.1A). A representative FPLC profile of ad lib 'true' triglycerides (Fig. 1B) indicated that glycerol interference does not account for the differences between genotype. Triglycerides clearly fall upon fasting in both genotypes. Two way ANOVA indicated that the reduction in triglycerides in 11βHSD-1^{-/-} mice upon fasting is significantly smaller in magnitude compared to wild type (Fig 1A). However, whilst wild-type triglyceride levels returned to ad lib fed values by 24 hours of re-feeding, 11βHSD-1^{-/-} triglyceride values returned to ad lib values by 4 hours and exhibited an overshoot to levels significantly higher than the ad lib fed group at 24 hours. Total and HDL cholesterol did not vary significantly with dietary manipulation (Fig. 2A and 2B). However, there is a highly significant effect of genotype, with 11βHSD-1^{-/-} mice having higher HDL cholesterol levels (~130% of wild type; Fig. 2B). Plasma glucose levels are similar in both genotypes in the fed state (wild type 6.24±0.04 versus null 5.8±0.5 mmol/L), with a trend towards lower fasting glucose in 11βHSD-1^{-/-} mice (wild type 4.04±0.3 versus null 3.4±0.1 mmol/L), as previously observed (16). Four hours re-fed glucose levels are similar (wild type 5.37±0.45 versus null 5.51±0.29 mmol/L), however, there is a small but significant decrease in 11βHSD-1 null glucose levels at 24 hours re-fed after a fast (wild type 5.51±0.45 versus null 4.64±0.15 mmol/L, p<0.05). This could reflect increased glucose tolerance in the 11βHSD-1^{-/-} mice. Plasma insulin is highly variable but similar in all feeding states in the 2 genotypes.

*Liver transcript profile of Fed 11βHSD-1*^{*-*/}*⁻ indicates normal lipid synthesis and increased lipid oxidation-* To investigate the origins of the alterations in plasma lipids, expression of mRNAs encoding enzymes involved in the lipid synthetic (Fig. 3) and fatty acid oxidation pathways (Fig. 4) are examined by northern blot analysis. Fatty acid synthase (FAS) (Fig 3A) and glycerol-phosphate acyl transferase (GPAT) (Fig 3B), enzymes involved in triglyceride synthesis and esterification, respectively, are similarly expressed in 11βHSD-1^{-/-} and wild-type mice under *ad lib* fed conditions. Indeed levels of the crucial lipogenic transcription factor SREBP-1c that drives expression of FAS, GPAT and other enzymes in the lipid synthesis pathway (25, 26) are comparable between genotypes (Fig 3C). This implies that reduced triglyceride synthesis and esterification is unlikely to play a role in the lowered plasma triglyceride profile of 11βHSD-1^{-/-} mice. Furthermore, mRNA encoding the rate-limiting enzyme in cholesterol synthesis, hydroxy-methyl-glutaryl-CoA-reductase (HMG-CoAR) is also expressed at similar levels in both genotypes in the fed state (Fig 3D).

In contrast, when enzymes of fatty acid oxidation are examined we found that mRNAs encoding carnitinepalmitoyl-transferase-I (CPT-1), a key rate-limiting enzyme in the mitochondrial β-oxidation pathway (27), acyl-CoA oxidase (ACO), a microsomal enzyme involved in fatty acid oxidation (28), and uncoupling protein-2 (UCP-2), a protein also implicated in the oxidation of fatty acids (29) and known to be expressed in hepatocytes (30), are all elevated in livers of fed 11 βHSD-1^{-/-} mice (Figs 4A, 4B, 4C). Moreover, PPARα mRNA, the key hepatic transcription factor regulating these genes of fatty acid oxidation is elevated in fed 11βHSD-1^{-/-} mice (Fig 4D). Elevated expression of CPT-I, ACO and UCP-2 is consistent with these genes being downstream targets of PPARα, (30-33).

### Example 2

### 11βHSD-1^{-/-} mice have an atheroprotective lipoprotein and fibrinogen profile

We also investigated the expression of glucocorticoid sensitive lipoproteins to further dissect the origin of the reduced triglyceride and increased HDL levels. Nephelometry is performed with specific anti-apolipoprotein antibodies on a representative sample of serum from both genotypes in the fed state. Consistent with a cardioprotective reduction in circulating triglycerides, serum levels of apoCIII, a triglyceride-rich component of VLDL that plays a key role in determining plasma triglyceride levels (34), is markedly reduced in 11βHSD-1^{-/-} mice (wild type 0.87±0.14 versus null 0.48±0.1 g/L). Apolipoprotein AI mRNA, encoding the major component of the HDL particle (35), is significantly elevated in fed 11βHSD-1^{-/-} mouse liver (Fig. 5A), with elevated circulating plasma apoAI levels. Interestingly, serum apoAII, another lipoprotein associated with the HDL particle is reduced (wild type 0.53±0.1 versus null 0.28±0.1 g/L). Serum levels of apoB and apoE are not different between genotypes.

To assess a hepatic transcript unrelated to lipoproteins or lipid metabolism, we investigated Aα-fibrinogen mRNA, which encodes a glucocorticoid-sensitive plasma factor (36) that is an independent cardiovascular risk factor (37). Aα-fibrinogen transcript levels are reduced by 25% in fed 11βHSD-1^{-/-} mice (Fig. 5B).

### Example 3

### 11βHSD-1^{-/}⁻ mice show attenuated induction of glucocorticoid-sensitive transcripts with fasting

Fasting causes a 2 fold induction of PPARα in wild type mice (Fig. 4D), consistent with reports that this transcription factor mediates glucocorticoid-induced fatty acid oxidation during fast (20, 21). However, whilst 11βHSD-1^{-/-} liver PPARα, levels are higher than wild type levels during *ad lib* fed conditions, fasting induction of PPARα mRNA is abolished in 11βHSD-1^{-/-} animals (Fig 4D). Despite the abolished induction of PPARα, the downstream target genes ACO and UCP-2 showed a fasting induction. This induction is smaller relative to the wild type ad lib to fasting induction. Such a modest induction could reflect the presence of relatively elevated ad lib fed PPARα levels in mice being activated by the increased levels of endogenous PPARα activators, fatty acids, during fasting. The glucocorticoid-inducible transcript apoAI also shows an attenuated rise on fasting, compatible with reduced effective glucocorticoid levels in hepatocytes (Fig 5B).

In agreement with an attenuated fasting response, a blunted fast-mediated repression of the lipid esterification enzyme GPAT is observed in null mice compared to wild type mice (Fig. 3B). Also, fasting induction of CPT-I (Fig. 4A) appears normal and fasting plasma glucose is not significantly different between genotypes. This implies that the attenuation of glucocorticoid effects on fatty acid oxidation and gluconeogenesis is not dramatic enough to cause hypoglycaemia after a 24 hour fast in the 11 βHSD-1^{-/-} mice.

*11βHSD-1 does not respond acutely to fasting*/*re-feeding in wild-type mice -* To determine that the difference between wild type and 11 βHSD-1^{-/-} mice are not merely due to feeding-related alterations in 11 βHSD-1 activity, transcript levels and activity of the wild type 11βHSD-1 is measured across the experimental groups. Neither 11βHSD-1 mRNA or activity levels are affected by a 24 hour acute fast or subsequent re-feeding (Fig. 7A, 7B). Thus, whilst the enzyme is critical for regulating the active intracellular glucocorticoid level, it does not appear to be acutely regulated by either the increased corticosterone (wild type, ad lib fed 25.2±7.2 versus wild type fasting 222±76 nmol/L, p< 0.05). Further, 11βHSD-1 mRNA and activity is not affected by the reduced insulin levels associated with fasting (wild type, ad lib 3131±81 versus wild type fasting 564±36 ng/ml) or with the subsequent influx of insulin upon re-feeding (4 hour re-fed value 6052±654 ng/ml).

### Example 4

### 11βHSD-1 mice have increased hepatic insulin sensitivity upon re-feeding after fast

We have investigated hepatic insulin sensitivity by assessing the relative changes in insulin-sensitive transcript levels upon re-feeding after a 24 hour fast. Northern analysis shows that insulin repressible transcripts such as CPT-1 and UCP-2 are more markedly suppressed in 11βHSD-1^{-/-} mice (Fig 4A and 4C) upon re-feeding. Conversely, insulin-inducible transcripts, such as those in the lipogenic (SREBP-1, FAS, GPAT) and cholesterologenic (HMG-CoAR) pathways, are more markedly induced in 11βHSD-1^{-/-} mice upon re-feeding (Fig 3A-D).

*11βHSD-1 mice have improved glucose tolerance -* Studies of dynamic glucose disposal indicate that 11 βHSD-1^{-/-} mice have improved glycaemic control (Fig. 8). Taking into account the reduced zero-time glucose levels in the 11 βHSD-1^{-/-} mice after fasting which likely reflects the attenuated stress reaction in fasting glucose production (16), area under the curve for glucose levels in 11βHSD-1^{-/-} mice indicates overall improved glucose disposal after an intraperitoneal glucose load compared to wild type. This is in keeping with improved hepatic insulin sensitivity.

### Example 5

### Effect of carbenoxolone on lipid profile in humans

Figure 6 shows the effect of administration of carbenoxolone on fasting plasma lipids in healthy humans and patients with type 2 diabetes mellitus.

6 men with type 2 diabetes mellitus and 6 healthy controls were administered placebo (filled bars) and carbenoxolone (open bars) in a randomised double-blind crossover study, as known in the art. Fasting levels of plasma lipids are shown. Carbenoxolone did not affect total cholesterol, but lowered triglyceride and raised HDL (high density lipoprotein) cholesterol concentrations.

### Example 6

### Effect of carbenoxolone on lipid profile in Zucker rats

The effects of inhibiting 11 β-HSD1 are determined in obese Zucker rats, to establish the metabolic effects of *in vivo* pharmacological manipulation of 11β-HSD1, and to assess the importance of tissue-specific changes in 11β-HSD1 activity on the therapeutic response in obesity. Carbenoxolone, a derivative of liquorice which inhibits both isozymes of 11β-HSD *in vivo* (68; 69), was used in these assays.

### Obesity

Vehicle treated obese Zucker rats had higher food consumption and gained more weight in the three-week treatment period than lean animals (Figure 13). Carbenoxolone treatment had no effect on food intake or body weight in either lean or obese animals.

### Glucose tolerance

In vehicle treated rats, obese animals had relative hyperglycemia and hyperinsulinaernia both on fasting and after glucose (Figure 9). Carbenoxolone treatment had no significant effect on plasma glucose in either group. By contrast, carbenoxolone increased plasma insulin in the fasting state and 30min after glucose in both lean and obese animals, and also at 120min in obese animals.

### Non-fasting plasma lipid levels

Total cholesterol was higher in obese than in lean rats, but was not affected by carbenoxolone (Figure 10). By contrast, HDL cholesterol was not different between lean and obese rats, and was increased by carbenoxolone in obese animals. Triglycerides were higher in obese than in lean rats, and were reduced by carbenoxolone treatment in obese animals. Non-fasting plasma NEFAs were not different between any of the groups.

### 11β-HSD activities in vitro

Amongst vehicle treated rats, tissue specific dysregulation of 11β-HSD1 activity in obesity was confirmed (), such that obese animals had lower activity in liver but higher activity in omental adipose tissue (Figure 11). Carbenoxolone administration resulted in similar measurable ex vivo inhibition of hepatic 11β-HSD 1 and renal 11β-HSD2 activities in lean and obese animals.

### Hypothalamic Pituitary Adrenal (HPA) axis

In vehicle treated rats, adrenal weight was higher in obese animals than in lean (Figure 13). Carbenoxolone treatment had no effect on adrenal weight in lean animals, but ameliorated adrenal hypertrophy in obese rats.

Plasma corticosterone levels were variable, probably reflecting uncontrolled stress at the time of decapitation. There were no statistically significant differences in plasma corticosterone levels, but there was a trend for plasma corticosterone to be higher in obese than lean animals (Figure 13) and for carbenoxolone to increase plasma corticosterone in both groups.

### 5β-Reductase activity in vitro

Glycyrrhetinic acid, from which carbenoxolone is derived, has been reported to inhibit other steroid metabolising enzymes, including 5β-reductase (70). 5β-Reductase irreversibly reduces the A-ring of glucocorticoids, thus inactivating them. Hepatic 5β-reductase activity was higher in obese animals than lean (Figure 12). Carbenoxolone, rather than inhibiting 5β-reductase, exacerbated the increase in obese animals.

This experiment assessed the efficacy of carbenoxolone in animals with the characteristic tissue-specific dysregulation of 11β-HSD1 that occurs in obesity (4; 3; 54). Obesity in these animals is confirmed to be associated with increased 11β-HSD1 in adipose tissue and down-regulation in liver. As in lean rats, carbenoxolone was effective in inhibiting 11β-HSD1 activity in liver and 11β-HSD2 activity in kidney. This illustrates that further reduction in hepatic 11β-HSD1 activity can be achieved pharmacologically in obese animals, beyond their basal down-regulation of enzyme expression and activity. Carbenoxolone had no effect on fasting plasma glucose or glucose tolerance. However, in the obese rats carbenoxolone did induce the same pattern of altered lipid profile (with decreased triglycerides and increased HDL cholesterol) which has been observed in the 11β-HSD1 knockout mouse (55). In the mouse model, this has been attributed to enhanced hepatic lipid oxidation rather than altered adipose metabolism, and probably results from up-regulation of PPARα in liver (55). A further lesson from the 11β-HSD1 knockout mouse is that differences in hepatic glucose metabolism were elicited only during dynamic testing (fasting and overfeeding)(16) while differences in lipid profile were more readily apparent. It may be that dynamic tests of hepatic glucose metabolism would reveal more subtle effects of carbenoxolone in the liver.

The HPA axis is activated in obese Zucker rats, and adrenocortical hypertrophy and hypercorticosteronaemia have been consistent findings (71). The adrenal hypertrophy but not the hypercorticosteronaemia were ameliorated by carbenoxolone in this experiment (Figure 13). This is most readily explained by the inhibition of renal 11 β-HSD2 inactivation of corticosterone, resulting in compensatory down-regulation of glucocorticoid secretion, as has been observed in humans given carbenoxolone (68).

In summary, these data show that inhibition of 11β-HSD1 with carbenoxolone in liver has beneficial effects on lipid metabolism in Zucker obese rats, despite lower basal 11β-HSD1 'target' activity.

### Example 7

### Liver-specific 11βHSD-1 expression

11β-HSD1, amplifying GC levels, is predominantly expressed in liver, adipose tissue and brain. Gene targeting studies in mice have revealed a requirement for 11β-HSD1 in induction of gluconeogenesis in the liver on stress or obesity (16). 11β-HSD *null* animals exhibit enhanced glucose tolerance and reduced plasma triglycerides (55). Adipose-specific over-expression of 11β-HSD1 in transgenic mice promotes central obesity, hyperlipidemia and insulin-resistant diabetes (49), but also increase GC delivery to the liver via the portal vein. To determine the metabolic consequences upon elevation of hepatic intracellular GC content alone we generated transgenic mice over-expressing 11β-HSD1 specifically in liver under the control of the Apo-E promoter.

A cDNA for rat 11β-HSD1 (with the open reading frame fused at the 3' end to an influenza virus haemagglutinin epitope (HA) tag) was inserted into a plasmid vector (86) previously used to direct liver-specific expression of a transgene in mice (87). Hepatic expression was driven by 3 kb of 5' flanking sequence plus a first exon and intron and part of a second exon from the human *apoE* gene upstream of the cDNA inserted. The sequence also included part of a final exon and a 770 bp enhancer downstream with transcription termination supplied by a SV40 polyadenylation signal. A ~5.5kb fragment was excised from this plasmid by NotI/partial *Eco*RI digestion and injected into the pronucleus of F1 CBA/C3H embryos for the generation of transgenic mice.

The results are shown in Figure 15. These lines exhibit at least 5-fold more 1113-HSD1 activity in the liver than wild types. Analyses show male mice have normal glucose tolerance, suggesting that hepatic 11β-HSD1 is not limiting for glucose homeostasis under basal conditions. However, transgenic animals show increased plasma insulin levels (~50%), elevated plasma triglycerides (~2 fold) and increased plasma total cholesterol (20%) largely attributable to the non-HDL fraction (HDL-cholesterol/total = 0.62 transgenic vs 0.83 wild type). Histological analyses reveal accumulation of lipid in hepatocytes of transgenic mice, suggesting lipid metabolism may be differentially sensitive to increased GC regeneration in the liver. In addition, male transgenic mouse body weight is modestly elevated compared to wild type littermates (6-8%), evident from ~10 weeks of age. The data indicate that selective increases in liver 11β-HSD1 modestly reduce insulin sensitivity, disadvantageously elevate plasma lipids and produce hepatic lipid accumulation, thus manifesting some, but not all aspects of the metabolic syndrome. The interplay between liver and adipose GC levels, determined by local 11β-HSD1, appear crucial in determining the manifestations of the Metabolic Syndrome.

### Example 8

### 11βHSD-1 influences metabolic balance

Transgenic mice overexpressing 11βBHSD-1 in adipose tissue under the control of the aP2 promoter (49) display a phenotype of hyperphagia, consuming a higher amount of food than wild-type mice, especially during high-fat feeding.

In 11βHSD-1 knockout mice (see Figure 16) hyperphagia is also observed at the onset of high fat feeding. However, body weight does not increase as a result of hyperphagia in these animals.

The lack of increase in body weight is attributable to an increase in metabolic rate observed in 11βHSD-1 knockout animals (-/-) compared to wild type controls (+/+). Rectal temperature (°C) was taken after around 14 weeks of high fat (HF) feeding.
+/+ control diet: 37.1+/-0.2 °C (n=16), +/+ hf: 37.6+/-0.2 °C (n=15)
-/- control: 37.7+/-0.2 °C (n=12), -/- hf: 38.3+/-0.2 °C (n=15)
by 2-way ANOVA effect of genotype: P<0.01
effect of diet: P<0.05

Knockout (-/-) mice fed a high fat diet show a substantially increased metabolic rate, which explains the lack of increase in body weight in response to hyperphagia.

### Example 9

### Adipose and macrophage PPARγ is increased in 11βHSD-1^{-/}⁻ mice

In addition to upregulation of hepatic PPARα and hence predicted synergism with PPARα agonists, increased PPARγ expression has been shown in adipose tissue in 11HSD1 -/- mice. The data are shown in Figure 17. This predicts synergism with PPARγ agonists (such as thiazolidinediones) in their beneficial effects on insulin sensitivity, plasma lipid profile, and glucose tolerance. The benefits of thiazolidinediones are attenuated by weight gain; in combination with 11HSD1 inhibition enhanced metabolic rate acts to prevent weight gain.

*Effects on macrophage lipid handling.* A key player in atherogenic pathology is the foam cell, a macrophage with lipid accumulation within the atheromatous plaque. Recent data in the art shows that PPARγ agonists increase cholesterol uptake in macrophages but crucially, they have a greater effect to increase cholesterol efflux (75, 76). Up-regulation of PPARγ mRNA in macrophages from 11HSD1 -/- mice has been shown, as set forth in Figure 18. Without wishing to be bound by any particular theory, 11βHSD-1 inhibition in macrophages may reduce foam cell formation and cholesterol storage in macrophages, with beneficial effects on atherogenic pathology.

### Example 10

### Effects of 11βHSD-1 on intrahepatic fat content

Liver-specific overexpression of 11β-HSD1 in mice results in increased hepatic fat content.

Livers from ApoE-11β-HSD-1 transgenic mice on normal chow diets were taken, sectioned and stained with Oil RedO (55). Lipid accumulation was observed in the transgenic mice over-expressing 11β-HSD-1 selectively in the liver.

Ectopic fat accumulation in insulin sensitive tissues such as the liver and skeletal muscle is associated with tissue-specific insulin resistance. As shown herein, excessive reactivation of cortisone to cortisol by 11β-hydroxysteroid dehydrogenase type 1 (11β-HSD-1) characterises subjects with increased fat accumulation in the liver and hepatic insulin resistance independently of obesity.

The study was conducted in 19 non-diabetic and apparently healthy men. Anthropometric and body composition measurements are shown in Figure 14. The mean LFAT content was 5-fold higher in men with high LFAT. Groups were similar with respect to age, physical fitness (VO2max), and markers of alcohol intake (MCV 90±1 vs 88±1 fl, AST/ALT 0.81±0.08 vs. 1.04±0.09 high vs low LFAT). Both AST (38±4 vs 25±2 U/L, p<0.02) and ALT (53±9 vs 27±4 U/L, p<0.02) were significantly higher in men with high LFAT. All measures of obesity including body mass index, the W/H, and MRI measurements of visceral and subcutaneous and the visceral/subcutaneous ratio were similar in the two groups.

Fasting plasma glucose and glycosylated haemoglobin concentrations were comparable between the groups, while men with high LFAT had higher fasting serum insulin, 40 % higher fasting serum triglycerides, and higher 24-hour systolic blood pressure (Figure 14). Concentrations of HDL and LDL cholesterol and 24-hour diastolic blood pressure were not significantly different between the groups.

### Insulin action in vivo

*Insulin action on endogenous glucose production and disposal.* During the insulin infusion, serum insulin concentrations averaged 25±2 in the men with a high and 21±1 mU/l in those with a low LFAT (NS). The increment above basal averaged 16±2 and 15±1 mU/l, respectively (NS). Rates of basal endogenous glucose Rₐ and R_{d} were comparable between the groups (2.3±0.2 vs. 2.5±0.2 mg/kg·min or 102±6 vs. 111±6 mg/m²·min, low vs. high LFAT, respectively). During the last hour of the insulin infusion, endogenous glucose Rₐ was significantly less suppressed in the men with high (41±15 %) as compared to those with low (91±16 %) LFAT (Figure 19a). Rates of glucose R_{d} were not different during the last hour of hyperinsulinemia (3.0±0.3 vs. 3.1±0.3 mg/kg·min, respectively, NS).

*Insulin action on serum FFA concentrations.* Fasting serum FFA concentrations were comparable basally (662±62 vs 550±54 µmol/l, NS, high vs. low LFAT) and during the first 2 hours of the insulin infusion (Figure 19b). During the last hour of the insulin infusion, serum FFA remained 44 % higher in the group with a high LFAT (329±41 vs. 228±23 µmol/l in low LFAT, p<0.05).

*Cortisol secretion and metabolism* The serum cortisol concentrations after oral cortisone acetate are depicted in Figure 20. In univariate analysis, the concentrations differed significantly at 75 and 90 min. In ANOVA for repeated measures, both LFAT (p<0.05 for time x LFAT) and BMI (p<0.05 for time x BMI) were independent, but opposing, determinants of serum cortisol concentrations at these time points. BMI and LFAT together explained 57 % (p=0.001) of the variance in plasma cortisol (mean concentration at 75-90 min). The regression equation for the mean serum cortisol concentration (mean of 75 and 90 min) was as follows: cortisol (nmol/l) = (1098±180) - (29±7 x BMI (kg/m², p<0.01)) + (7.4±3.3 x LFAT (%, p<0.05)). Over the range of LFAT observed (1-23 %) at a constant BMI of 25 kg/m², the predicted variation in S-cortisol is 380 to 543 nmol/l, which would correspond to variation in BMI from 27 to 22 kg/m² at a constant LFAT % (10 %).

Men with ectopic fat accumulation in the liver exhibit selective hepatic insulin resistance and convert more cortisone to cortisol, suggesting enhanced hepatic 11β-HSD-1 activity. These data support the concept that excess tissue glucocorticoid action contributes to hepatic insulin resistance.

### Example 11

### Inhibition of 11b-HSD1 protects from adverse effects of synthetic glucocorticoids

Synthetic glucocorticoids can be used in anti-inflammatory therapy (e.g. in inflammatory bowel disease, arthritis, asthma) and are metabolised by 11β-HSD enzymes. Examples include prednisolone interconversion with prednisone (78) and dexamethasone interconversion with 11-dehydrodexamethasone (77). As a result, inactive 11-keto-steroid is in the circulation during treatment with these drugs and may be reactivated to active 11-hydroxy-steroid in tissues where 11β-HSD1 is expressed. Thus, inhibition of 11β-HSD1 will prevent reactivation of glucocorticoid in these sites and may protect against undesired adverse effects of glucocorticoids, allowing targeting of glucocorticoid action to sites where the anti-inflammatory effects are desirable.

The manipulation of glucocorticoid effects of the synthetic glucocorticoid beclomethasone is demonstrated. Beclomethasone is subject to metabolism by 11β-HSD1. Figure 21 a shows incubation of beclomethasone with homogenised rat liver in conditions known in the art (82) containing 11β-HSD1 which in homogenised conditions functions as a dehydrogenase. Conversion of beclomethasone to 11-dehydrobeclomethasone was measured by HPLC with on-line ultraviolet detection at 254 nm. The conversion observed during this incubation confirms that beclomethasone is a substrate for 11β-HSD1.

Beclomethasone is a glucocorticoid receptor agonist. It is also demonstrated that inhibition of 11β-HSD1 reduces a glucocorticoid receptor-mediated response in human skin (79). 12 healthy humans aged 20-33 years had beclomethasone applied to the skin of the forearm, according to Noon et al 1996. On separate areas of skin, beclomethasone was applied together with glycyrrhetinic acid, an 11β-HSD inhibitor (81). The intensity of skin blanching was recorded the following day by two observers who were blind to the order of application of steroid solutions, as described (80). Total scores were calculated for the area under the dose-response curve for beclomethasone or for beclomethasone with the addition of glycyrrhetinic acid. Results are shown in Figure 21b. Glycyrrhetinic acid reduced the blanching response to beclomethasone, which without being bound to any particular theory suggests that prevention of reactivation of beclomethasone within the skin by 11β-HSD1 attenuates the local glucocorticoid potency.

### REFERENCES

1. Peeke, P.M. and Chrousos, G.P. (1995) Ann. N. Y. Acad. Sci. 771, 665-676.
2. Andrew, R., Phillips, D.I.W. and Walker, B.R. (1998) J. Clin. Endocrinol. Metab. 83, 1806-1809.
3. Stewart, P.M., Boulton, A., Kumar, S., Clark, P.M.S. and Shackleton, C.H.L. (1999) J. Clin. Endocrinol. Metab. 84, 1022-1027.
4. Livingstone, D.E., Jones, G.C., Smith, K., Jamieson, P.M., Andrew, R, Kenyon, C.J. and Walker, B.R. (2000) Endocrinol. 141, 560-563.
5. Brown, R.W., Chapman, K.E., Edwards, C.R.W. and Seckl, J.R. (1993) Endocrinol. 132, 2614-2621. Human Placental 1 1b-hydroxysteroid dehydrogenase:
6. Albiston A.L., Obeyesekere, V. R., Smith, R.E., Krozowski, Z.S. (1994) Mol. Cell. Endo. 105, R11-R17.
7. Edwards, C.R.W., Stewart, P.M., Burt, D., Brett, L., Mclntyre, M.A., Sutanto, W.S. de Kloet, E.R. and Monder, C. (1988) Lancet Oct 29;2(8618):986-989.
8. Funder, J.W., Pearce, P.T., Smith, R. and Smith, A.I. (1988) Science 242, 583-585
9. Bujalska, I.J., Kumar, S. and Stewart, P.M. (1997) Lancet 349 (9060) 1210-1213. Does central obesity reflect Cushing's disease of the omentum?
10. Agarwal, A.K., Monder, C., Eckstein, B. and White, P.C. (1989) J. Biol. Chem. 264, 18939-18943.
11. Rajan, V., Chapman, K.E., Lyons, V., Jamieson, P.M., Mullins, J.J., Edwards, C.R.W. and Seckl, J.R. (1995) J. Steroid. Biochem. Molec. Biol. 52, 141-147.
12. Jamieson, P.M., Chapman, K.E., Edwards, C.R.W. and Seckl, J.R. (1995) Endocrinol. 136,4754-4761.
13. Napolitano, A., Voice, M., Edwards, C.R.W., Seckl, J.R. and Chapman, K.E. (1998) J. Steroid Biochem. Molec. Biol. 64, 251-260.
14. Rajan, V., Edwards, C.R.W. and Seckl, J.R. (1996) J. Neurosci. 16, 65-70.
15. Jamieson, P.M., Walker, B.R. Chapman, K.E., Andrew, R., Rossiter, S. and Seckl, J.R. (2000) J. Endocrinol. 165, 685-692. 11
16. Kotelevtsev, Y., Holmes, M.C., Burchell, A., Houston, P.M., Schmoll, D., Jamieson, P., Best, R., Brown, R., Edwards, C.R.W., Seckl, J.R. and Mullins, J.J. (1997) Proc. Natl. Acad. Sci. USA 94, 14924-14929.
17. Grundy, S.M. (1998) Am. J. Cardiol. 81, 18B-25B.
18. Lemberger, T, Staels, B, Saladin, R., Desvergne, B., Auwerx, J. and Wahli, W. (1994) J. Biol. Chem. 269, 24527-24530.
19. Lemberger, T., Saladin, R., Vasquez, M., Assimacopoulos, F., Staels, B., Desvergne, B., Wahli, W and Auwerx, J. (1996) J. Biol. Chem. 271, 1764-1769.
20. Kersten, S., Seydoux, J., Peters, J.M., Gonzalez, F.J., Desvergne, B. and Wahli, W. (1999) J. Clin. Invest. 103, 1489-1498.
21. Leone, T.C., Weinheimer, C.J. and Kelly, D.P (1999) Proc. Natl. Acad. Sci. USA 96, 7473-7478.
22. Krey, G., Braissant, O., L'Horset, F., Kalkhoven, E., Perroud, M., Parker, M.G. and Wahli, W. (1997) Mol. Endocrinol. 11, 779-791.
23. Peters, J.M., Hennuyer, N., Staels, B., Fruchart, J.-C., Fievet, C., Gonzalez, F.J. and Auwerx, J. (1997) J. Biol. Chem. 272, 27307-27312.
24. Harris, H.J., Kotelevtsev, Y., Mullins, J.J., Seckl, J.R. and Holmes, M.C. (2001) Endocrinology 2001 142: 114-120
25. Foretz, M., Guichard, C., Ferre, P. and Foufelle, F. (1999) Proc. Nail. Acad. Sci USA 96, 12737-12742.
26. Shimano, H., Yahagi, N., Amemiya-Kudo, M., Hasty, A.H., Osuga, J.-I., Tamura, Y., Shionoiri, F., Iizuka, Y., Ohashi, K., Harada, K., Gotoda, T., Ishibashi, S and Yamada, N. (1999) J. Biol. Chem. 274, 35832-35839.
27. McGarry, J.D. and Brown, N.F. (1997) Eur. J. Biochem. 244, 1-14.
28. Fan, C.Y., Pan, J., Usuda, N., Yeldandi, A.V., Rao, M.S. and Reddy, J.K. (1998) J. Biol. Chem. 273, 15639-15645.
29. Boss, O., Hagan, T. and Lowell, B.B. (2000) Diabetes 49, 143-156.
30. Kelly, L.J., Vicario, P.P., Thompson, G.M., Candelore, M.R., Doebber, T.W., Ventre, J., Wu, M.S., Meurer, R., Forest, M.J., Conner, M.W., Cascieri, M.A. and Moller, D.E. (1998) Endocrinol. 139, 4920-4927.
31. Yu, G.S., Lu, Y.C. and Gulick, T. (1998) J. Biol. Chem. 273, 32901-32909.
32. Lee, S.-S., Pineau, T., Drago, J., Lee, E.J., Owens, J.W., Kroetz, D.L., Fernandez-Salguero, P.M., Westphal, H. and Gonzalez, F.J. (1995) Mol. Cell. Biol. 15,3012.
33. Tsuboyama-Kasaoka, N., Takahashi, M., Kim, H. and Ezaki, O. (1999) Biochem Biophys. Res. Commun. 257, 879-885.
34. Ebara, T., Ramakrishnan, R., Steiner, G. and Shachter, N.S., (1997) J. Clin. Invest. 99, 2672-2681.
35. Barter, P.J. and Rye, K.A. (1996) Atherosclerosis 121, 1-12.
36. Bhattacharya, A and Holland, L.J. (1991) Mol. Endocrinol. 5(4), 587-597.
37. Kannel, W.B. (1997) Drugs 54, Suppl 3, 32-40.
38. Aalto-Setala, K., Fisher, E.A., Chen, X., Chajek-Shaul, T., Hayek, T., Zechner, R., Walsh, A., Ramakrishnan, R., Ginsberg, H.N. and Breslow, J.L. (1992) J. Clin. Invest. 90, 1889-1890.
39. Ito, Y., Azrolan, N., O'Connell, A., Walsh, A. and Breslow, J.L. (1990) Science 249, 790-793..
40. Dammerman, M., Sankuijl, L.A., Halaas, J.L., Chung, W. and Breslow, J.L. (1993) Proc. Natl. Acad Sci USA 90, 4562-4566.
41. Kockx, M., Gervois, P.P., Poulain, P., Derudas, B., Peters, J.M., Gonzalez, F.J., Princen, H.M., Kooistra, T and Staels, B. (1999) Blood 93, 2991-2998.
42. Yau et al., (2001) Proc. Natl. Acad. Sci. USA, 98,4716-4721.
43. Cole, T.J., Harris, H.J., Hoong, I., Solomon, N., Smith, R., Krozowski, Z. and Fullerton, M.J. (1999)
44. Dong, Y., Poellinger, L., Gustafsson, J.A. and Okret, S. (1988) Mol. Endocrinol. 2(12), 1256-1264. Collett, G.P., Betts, AM., Johnson, M.I., Pulimood, A.B., Cook, S., Neal, D.E. and Robson C.N. (2000) Clin. Cancer Res. 6,3241-3248.
45. Qi, C., Zhu, Y.J. and Reddy, J.K.(2000)Cell Biochem. Biophys 32, 187-204.Valmaseda, A, Carmona, M.C., Barbera, M.J., Vinas, O., Mampel, T., Iglesias, R., Villarroya, F., Giralt, M.. 1(999) Mol. Cell. Endocrinol. 154, 101-109.
46. Schultz R., Yan, W., Toppari, J., Volkl, A., Gustafsson, J.A. and Pelto-Huikko, M. (1999) Endocrinol. 140, 2968-2975.
47. Ambrosi, B., Sartorio, A., Pizzocaro, A., Passini, E., Bottasso, B. and Federici, A. (2000) Exp.Clin. Endocrinol. Diab. 108, 294-298.
48. Staels, B., van Tol, A., Chan, L., Verhoeven, G. and Auwerx, J., (1991) Arterioscler Thromb. May-Jun;11 (3):760-9.
49. Masuzaki et al., (2001) Science 294:2166-2170
50. Kim JK, Gavrilova O, Chen Y, Reitman ML, Shulman GI. J Biol Chem 2000; 275: 8456-8460.
51. Gavrilova O, Marcus-Samuels B, Graham D, et al. J Clin Invest 2000; 105: 271-278.
52. Ryysy L, Hakkinen AM, Goto T, et al. Diabetes 2000; 49: 749-758.
53. Marchesini G, Brizi M, Bianchi G, et al. Diabetes 2001; 50: 1844-1850.
54. Rask E, Olsson T, Soderberg S, Andrew R, Livingstone DEW, Johnson O & Walker BR 2001 Journal of Clinical Endocrinology and Metabolism 86 1418-1421.
55. Morton NM, Holmes MC, Fievet C, Staels B, Tailleux A, Mullins JJ & Seckl JR 2001 Journal of Biological Chemistry 276 41293-41300.
56. Stewart PM, Wallace AM, Atherden SM, Shearing CH, Edwards CR. Clin Sci (Colch) 1990; 78: 49-54.
57. Best R, Walker BR. Clin Endocrinol (Oxf) 1997; 47: 231-236.
58. DeFronzo RA, Tobin JD, Andres R. Am J Physiol 1979; 237: E214-E223
59. Puhakainen I, Koivisto VA, Yki-Järvinen H. Diabetes 1991; 40: 1319-1327.
60. Steele R. Ann NY Acad Sci 1959; 82: 420-430.
61. Thomsen C, Becker U, Winkler K, Christoffersen P, Jensen M, Henriksen O. Magn Reson Imaging 1994; 12: 487-495.
62. Stenman U-H, Pesonen K, Ylinen K, Huhtala ML, Teramo K. J Chromatog 1984; 297: 327-332.
63. Kadish AH, Little RL, Sternberg JC. Clin Chem 1968; 14: 116-131.
64. Yki-Järvinen H, Kauppila M, Kujansuu E, et al. N Engl J Med 1992; 327: 1426-1433.
65. Desbuquois B, Aurbach GD. J Clin Endocrinol Metab 1971; 33: 732-738.
66. Miles J, Classcock R, Aikens J, Gerich J, Haymond N. J Lipid Res 1983; 24: 96-99.
67. Lukaski HC, Johnson PE, Bolonchuk WW, Lykken GI. Am J Clin Nutr 1985; 41: 810-817.
68. Stewart PM, Wallace AM, Atherden SM, Shearing CH & Edwards CRW 1990 Clinical Science 78 49-54.
69. Jellinck PH, Monder C, McEwen BS & Sakai RR 1993 Journal of Steroid Biochemistry and Molecular Biology 46 209-213.
70. Latif SA, Conca TJ & Morris DJ 1990 Steroids 55 52-58.
71. Bestetti GE, Abramo F, Guillaume-Gentil C, Rohner-Jeanrenaud F, Jeanrenaud B & Rossi GL 1990 Endocrinology 126 1880-1887.
72. Tannin, et al. (1991) J. Biol. Chem. 266: 16653-16658.
73. Studier et al, (1990) Methods in Enzymol. 185; 60-89.
74. Al-Dujaili et al. (1981) Steroids 37 157-176.
75. Chawla et al. (2001) Nature Medicine, 7:48-52.
76. Chinetti et al. (2001) Nature Medicine, 7:53-58.
77. Best et al. (1997) Journal of Endocrinology 153, 41-48.
78. Conti et al. (1994) Nephrology, Dialysis, Transplantation 9, 1622-1628.
79. Marks et al. (1982) Journal of Clinical Endocrinology and Metabolism 54, 1075-1077.
80. Noon et al. (1996) British Journal of Dermatology 134, 837-842.
81. Teelucksingh et al. (1990) Lancet 335, 1060-1063.
82. Walker et al. (1992). Endocrinology 131, 970-972.
83. Le May et al. (2000) FEBS Letters 475, 163-166.
84. Forman et al. (1997) PNAS 94, 4312-4317.
85. Murao et al. (1998) Journal of Biological Chemistry 273, 18959-18965.
86. Fan et al. (1994) PNAS 91, 8724-8728.
87. Wang et al. (1998) J. Biol. Chem. 273, 32920-32926.

## Claims

1. Use of an agent which reduces intracellular 11β-HSD1 activity and a PPARα agonist in the manufacture of a composition for the promotion of an atheroprotective lipid profile.

2. Use according to claim 1, wherein 11β-HSD1 levels are lowered by an agent which modulates the expression of the endogenous 11β-HSD1 gene.

3. Use according to claim 1 or claim 2, wherein 11β-HSD1 levels are lowered by an agent which modulates 11β-HSD1 mRNA, transcription or translation.

4. Use according to claim 1 or claim 2, wherein 11β-HSD1 levels are lowered by an agent which inhibits 11β-HSD1 synthesis or activity.

5. Use according to claim 4, wherein said agent is selected from the group consisting of carbenoxolone, 11-oxoprogesterone, 3α,17,21-trihydoxy-5β-pregnan-3-one, 21-hydroxy-pregn-4-ene-3,11,20-trione, androst-4-ene-3,11,20-trione and 3β-hydroxyandrost-5-en-17-one.

6. Use according to any preceding claim, wherein the atheroprotective lipid profile comprises a reduction in plasma triglyceride levels.

7. Use according to any preceding claim, wherein the atheroprotective lipid profile comprises an increase in HDL cholesterol levels.

8. Use according to any preceding claim, wherein serum apoCIII levels are reduced as a consequence of the reduction of 11 β-HSD 1 levels.

9. A pharmaceutical composition comprising an agent which reduces 11β-HSD1 activity and a PPARα agonist.

10. An agent which reduces 11β-HSD1 activity and a PPARα agonist for simultaneous, simultaneous separate or sequential use in the promotion of an atheroprotective lipid profile.

11. A kit comprising an agent which reduces 11β-HSD1 activity and a PPARα agonist, and instructions for use in the promotion of an atheroprotective lipid profile.

12. A kit comprising an agent which reduces 11β-HSD1 activity and a PPARα agonist, packaged in unit doses for use in the promotion of an atheroprotective lipid profile.

13. A pharmaceutical composition comprising an agent which reduces 11β-HSD1 activity and a PPARγ agonist.

14. An agent which reduces 11β-HSD1 activity and a PPARγ agonist for simultaneous, simultaneous separate or sequential use in the control of cardiovascular risk.

15. A kit comprising an agent which reduces 11β-HSD1 activity and a PPARγ agonist, and instructions for use in the control of cardiovascular risk.

16. A kit comprising agent which reduces 11β-HSD1 activity and a PPARγ agonist, packaged in unit doses for use in the control of cardiovascular risk.

## Patentansprüche

1. Verwendung eines Agens, welches die intrazelluläre 11β-HSD1-Aktivität reduziert, und eines PPARα-Agonisten bei der Herstellung einer Zusammensetzung für die Förderung eines atheroprotektiven Lipidprofils.

2. Verwendung gemäß Anspruch 1, wobei 11β-HSD1-Spiegel durch ein Agens herabgesetzt werden, das die Expression des endogenen 11β-HSD1-Gens moduliert.

3. Verwendung gemäß Anspruch 1 oder Anspruch 2, wobei 11β-HSD1-Spiegel durch ein Agens herabgesetzt werden, welches die Transkription oder Translation von 11β-HSD1-mRNA moduliert.

4. Verwendung gemäß Anspruch 1 oder Anspruch 2, wobei 11β-HSD1-Spiegel durch ein Agens herabgesetzt werden, welches die 11β-HSD1-Synthese oder -Aktivität inhibiert.

5. Verwendung gemäß Anspruch 4, wobei das Agens ausgewählt ist aus der Gruppe, bestehend aus Carbenoxolon, 11-Oxoprogesteron, 3α,17,21-Trihydroxy-5β-pregnan-3-on, 21-Hydroxypregn-4-en-3,11,20-trion, Androst-4-en-3,11,20-trion und 3β-Hydroxyandrost-5-en-17-on.

6. Verwendung gemäß einem der vorangehenden Ansprüche, wobei das atheroprotektive Lipidprofil ein Herabsetzen von Plasma-Triglycerid-Spiegeln umfaßt.

7. Verwendung gemäß einem der vorangehenden Ansprüche, wobei das atheroprotektive Lipidprofil ein Ansteigen von HDL-Cholesterin-Spiegeln umfaßt.

8. Verwendung gemäß einem der vorangehenden Ansprüche, wobei Serum-ApoCIII-Spiegel herabgesetzt werden, als eine Folge des Herabsetzens der 11β-HSD1-Spiegel.

9. Pharmazeutische Zusammensetzung, welche ein Agens umfaßt, das die 11β-HSD1-Aktivität herabsetzt, und einen PPARα-Agonisten.

10. Agens, welches die 11β-HSD1-Aktivität reduziert, und ein PPARα-Agonist zur simultanen, simultanen getrennten oder sequentiellen Verwendung bei der Förderung eines atheroprotektiven Lipidprofils.

11. Kit, welches ein Agens umfaßt, welches die 11 β-HSD1-Aktivität herabsetzt, und einen PPARα-Agonisten und Anweisungen für die Verwendung bei der Förderung eines atheroprotektiven Lipidprofils.

12. Kit, welches ein Agens umfaßt, welches die 11 β-HSD1-Aktivität herabsetzt, und einen PPARa-Agonisten, welches in Dosiseinheiten verpackt ist, zur Verwendung bei der Förderung eines atheroprotektiven Lipidprofils.

13. Pharmazeutische Zusammensetzung, welche ein Agens umfaßt, das 11β-HSD1-Aktivität herabsetzt, und einen PPARy-Agonisten.

14. Agens, welches 11β-HSD1-Aktivität herabsetzt, und ein PPARy-Agonist zur simultanen, simultanen getrennten oder sequentiellen Verwendung bei der Kontrolle von kardiovaskulärem Risiko.

15. Kit, welches ein Agens umfaßt, welches 11β-HSD1-Aktivität herabsetzt, und einen PPARy-Agonisten und Anweisungen für die Verwendung bei der Kontrolle von kardiovaskulärem Risiko.

16. Kit, welches ein Agens umfaßt, welches 11β-HSD1-Aktivität herabsetzt, und einen PPARγ-Agonisten, welches in Dosiseinheiten verpackt ist, zur Verwendung bei der Kontrolle von kardiovaskulärem Risiko.

## Revendications

1. Utilisation d'un agent qui réduit l'activité de 11β-HSD1 intracellulaire et d'un agoniste de PPARα dans la production d'une composition destinée à favoriser un profil lipidique athéroprotecteur.

2. Utilisation suivant la revendication 1, dans laquelle les taux de 11β-HSD1 sont réduits par un agent qui module l'expression du gène 11β-HSD1 endogène.

3. Utilisation suivant la revendication 1 ou la revendication 2, dans laquelle les taux de 11β-HSD1 sont réduits par un agent qui module la transcription ou la traduction de l'ARNm de 11β-HSD1.

4. Utilisation suivant la revendication 1 ou la revendication 2, dans laquelle les taux de 11β-HSD1 sont réduits par un agent qui inhibe la synthèse ou l'activité de 11β-HSD1.

5. Utilisation suivant la revendication 4, dans laquelle ledit agent est choisi dans le groupe consistant en la carbénoxolone, la 11-oxoprogestérone, la 3α,17,21-tri-hydroxy-5β-prégnane-3-one, la 21-hydroxy-prégn-4-ène-3,11,20-trione, l'androst-4-ène-3,11,20-trione et la 3β-hydroxy-androst-5-ène-17-one.

6. Utilisation suivant l'une quelconque des revendications précédentes, dans laquelle le profil lipidique athéroprotecteur comprend une réduction des taux plasmatiques de triglycérides.

7. Utilisation suivant l'une quelconque des revendications précédentes, dans laquelle le profil lipidique athéroprotecteur comprend une augmentation des taux de cholestérol-HDL.

8. Utilisation suivant l'une quelconque des revendications précédentes, dans laquelle les taux sériques de apoCIII sont réduits en conséquence de la réduction des taux de 11β-HSD1.

9. Composition pharmaceutique comprenant un agent qui réduit l'activité de 11β-HSD1 et un agoniste de PPARα.

10. Agent qui réduit l'activité de 11β-HSD1 et agoniste de PPARα pour une utilisation simultanée, séparée simultanée, ou séquentielle, pour favoriser un profil lipidique athéroprotecteur.

11. Kit comprenant un agent qui réduit l'activité de 11β-HSD1 et un agoniste de PPARα, et des instructions d'utilisation pour favoriser un profil lipidique athéroprotecteur.

12. Kit comprenant un agent qui réduit l' activité de 11β-HSD1 et un agoniste de PPARα, conditionnés en doses unitaires à des fins d'utilisation pour favoriser un profil lipidique athéroprotecteur.

13. Composition pharmaceutique comprenant un agent qui réduit l'activité de 11β-HSD1 et un agoniste de PPARγ.

14. Agent qui réduit l'activité de 11β-HSD1 et agoniste de PPARγ pour une utilisation simultanée, séparée simultanée, ou séquentielle, dans la lutte contre le risque cardio-vasculaire.

15. Kit comprenant un agent qui réduit l'activité de 11β-HSD1 et un agoniste de PPARγ, et des instructions d'utilisation à des fins d'utilisation pour lutter contre le risque cardio-vasculaire.

16. Kit comprenant un agent qui réduit l'activité de 11β-HSD1 et un agoniste de PPARγ, conditionnés en doses unitaires, à des fins d'utilisation pour lutter contre le risque cardio-vasculaire.
